# FASCICULE DE BREVET EUROPEEN

(11) **EP 4 153 172 B1**
(45) Date de publication et mention de la délivrance du brevet: **27.11.2024**
(21) Numéro de dépôt: 21725778.1
(22) Date de dépôt: 18.05.2021
(51) Int. Cl.: A61K 31/4184, A61K 31/4196, A61K 31/423, A61K 31/433, A61K 31/437, A61K 31/4406, A61K 31/498, A61K 31/519, A61K 31/52, A61P 31/18, A61P 43/00

(54) **COMPOSÉS POUR LEUR UTILISATION POUR LA RÉACTIVATION DU VIH DANS DES CELLULES LATENTES INFECTÉES PAR LE VIH**
VERBINDUNGEN ZUR VERWENDUNG BEI DER REAKTIVIERUNG VON HIV IN LATENTEN HIV-INFIZIERTEN ZELLEN
COMPOUNDS FOR USE IN THE REACTIVATION OF HIV IN LATENT HIV-INFECTED CELLS

(30) Priorité: 20.05.2020 FR 2005250
(43) Date de publication de la demande: 29.03.2023
(73) Titulaire: Centre National de la Recherche Scientifique, 75016 Paris (FR); UNIVERSITE DE MONTPELLIER, 34090 Montpellier (FR)
(72) Inventeur: BEAUMELLE, Bruno, 34160 SAINT DREZERY (FR); CHALOIN, Laurent, 34730 PRADES LE LEZ (FR)
(74) Mandataire: Cabinet Becker et Associés
(86) Numéro de dépôt international: PCT/EP2021/063206
(87) Numéro de publication internationale: WO 2021/233950

(56) Documents cités:
- US-A1- 2019 092 749
- PIRAS ET AL.: "DICHLORO-PHENYL-BENZOTRIAZOLES: A NEW SELECTIVE CLASS OF HUMAN RESPIRATORY SYNCYTIAL VIRUS ENTRY INHIBITORS", FRONTIERS IN CHEMISTRY, vol. 7, 247, 2019, pages 1 - 18, XP002801793
- KHUMOEKAE RICHARD ET AL: "Identification of Novel HIV-1 Latency-Reversing Agents from a Library of Marine Natural Products", VIRUSES, vol. 10, no. 7, 27 June 2018 (2018-06-27), pages 348, XP055763679, DOI: 10.3390/v10070348
- YANG HUNG-CHIH ET AL: "Small-molecule screening using a human primary cell model of HIV latency identifies compounds that reverse latency without cellular activation", THE JOURNAL OF CLINICAL INVESTIGATION : JCI, AMERICAN SOCIETY FOR CLINICAL INVESTIGATION, US, vol. 119, no. 11, 1 November 2009 (2009-11-01), pages 3473 - 3486, XP009159909, ISSN: 1558-8238, DOI: 10.1172/JCI39199
- NGUYEN WILLIAM ET AL: "Optimization of 5-substituted thiazolyl ureas and 6-substituted imidazopyridines as potential HIV-1 latency reversing agents", EUROPEAN JOURNAL OF MEDICINAL CHEMISTRY, ELSEVIER, AMSTERDAM, NL, vol. 195, 20 March 2020 (2020-03-20), XP086144022, ISSN: 0223-5234, [retrieved on 20200320], DOI: 10.1016/J.EJMECH.2020.112254

## Description

### Domaine technique

La présente invention a trait au traitement des maladies virales, et plus particulièrement au traitement des maladies liées au VIH. Plus précisément, l'invention a trait à des composés aptes à réactiver le VIH dans des cellules latentes infectées par le VIH.

### Arrière-plan technologique

Le virus de l'immunodéficience humaine, ou VIH, est un rétrovirus du genre lentivirus qui infecte l'homme et est responsable du syndrome d'immunodéficience acquise (SIDA). La propagation mondiale du VIH et le nombre extrêmement élevé de personnes infectées par le virus ont fait du SIDA une priorité mondiale en matière de santé.

L'infection par le VIH est une infection chronique avec une réplication virale continue conduisant à une diminution du nombre de lymphocytes T CD4 et à une immunodépression. La réplication virale peut être réduite par des médicaments antirétroviraux (ARTs). Les traitements antirétroviraux (ARTs) utilisent des petites molécules capables d'inhiber la réplication virale du VIH et regroupées en classe selon leur cible. La réduction de la réplication virale est suivie par une augmentation des lymphocytes T CD4.

Bien que les ARTs suppriment efficacement la réplication virale du VIH-1 en bloquant diverses étapes du cycle de vie du virus, les ARTs ne permettent pas de guérir l'infection en raison de l'existence de réservoirs à longue durée de vie. Il s'agit principalement de lymphocytes T-CD4⁺ quiescents qui contiennent un provirus VIH-1 intégré mais qui ne produisent pas de virus. L'expression des protéines au stade de latence est limitée. La cellule infectée est donc indifférenciée des cellules normales et n'est pas ciblée ni par l'ART ni par le système immunitaire. La latence est un stade de l'infection virale non productif, mais réversible. Ce sont ces cellules latentes qui vont permettre un rebond viral lors de l'interruption de l'ART. Ainsi, la charge virale redevient détectable chez la majorité des individus infectés après 2 semaines d'interruption du traitement ART. Les lymphocytes T infectés latents sont rares (1-100 par million de cellules T CD4⁺ selon les tests de croissance virale) mais ils ont une demi-vie extrêmement longue (44 mois). Ces cellules latentes constituent donc un obstacle majeur à l'éradication virale. En outre, ce réservoir de cellules latentes se met en place de façon dramatiquement précoce après l'infection : un patient infecté dix jours auparavant a déjà près de 200 cellules latentes dans l'organisme et ne pourra donc pas être "guéri" par des ARTs.

Une de stratégies visant à l'éradication de ce réservoir de VIH-1 nécessite l'activation de ces cellules infectées latentes (phase "Shock"), afin qu'elles puissent être tuées par le virus ou reconnues par des effecteurs immunitaires (phase "Kill"). C'est le paradigme "shock-and-kill".

L'activation du VIH-1 dans les cellules infectées latentes nécessite l'utilisation d'agents de levée ou de réversion de latence (LRAs). La plupart des LRAs disponibles visent des protéines cellulaires telles que les histone-désacétylases (HDAC), la protéine kinase C, les méthyl transférases ou le facteur d'élongation de transcription P-TEFb. Bien que ces LRAs, puissent activer la production virale dans des lignées cellulaires ou des cellules primaires hébergeant des virus latents, surtout lorsqu'ils sont utilisés en combinaison, aucun d'entre eux n'a jusqu'ici démontré un effet significatif sur la taille du réservoir viral chez les patients. En effet, ces LRAs ciblent des protéines cellulaires et ont de nombreux effets secondaires. En particulier, elles inhibent la fonction cytotoxique des cellules CD8, alors que celle-ci est cruciale pour la phase "Kill". Cet effet est probablement responsable de l'incapacité de ces molécules à diminuer la taille du réservoir chez les patients. Khumoekae et al., Viruses, 2018, vol. 10. 10, n. 7, 348 décrit certains LRAs.

Ainsi, il subsiste un besoin en composés sûrs, capables de réactiver efficacement le VIH dans des cellules latentes de patients infectés par le VIH.

### Résumé de l'invention

Dans ce contexte, les inventeurs ont identifié de nouveaux composés LRAs ciblant non plus des protéines cellulaires mais une protéine virale. Plus précisément, les composés selon l'invention activent la protéine Tat (« Trans-Activator of Transcription ») du VIH, qui en interagissant avec le domaine TAR (« Trans-Activation Responsive élément ») des séquences ARN viral, permet la transcription des gènes viraux. Le domaine TAR est composé des 57 premiers nucléotides communs à tous les ARN du VIH. Tat est une protéine virale clé qui est exprimée à faible taux dans les lignées de cellules latentes. Les composés selon l'invention présentent une forte affinité pour le complexe de transcription Tat-TAR, et favorisent l'activité transcriptionnelle de cette protéine, permettant ainsi de lever la latence du VIH dans les cellules infectées par le VIH.

L'invention a donc pour objet un composé selon la formule (I), ou un sel de celui-ci, 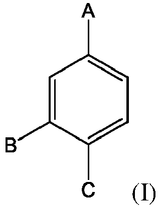 dans lequel
A représente un hétéroaryl optionnellement substitué par au moins un halogène, et
B et C représentent indépendamment l'un de l'autre, un radical choisi parmi un hydrogène, un alkyle en C₁-C₆, un alcoxy C₁-C₆, et un groupe selon la formule (II), 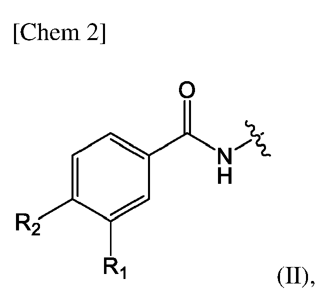
   dans laquelle R₁ et R₂ représentent indépendamment l'un de l'autre, un radical choisi parmi un hydrogène,
   un alkyle en C₁-C₃,
   un alcoxy en C₁-C₃,
   un groupe selon la formule -Y-[W]n-Z, dans lequel
   Y est un radical choisi parmi O, N, S et C, préférentiellement un radical choisi parmi O, N, et C
   W est un radical choisi parmi un alkyle en C₁-C₆,un alkylène en C₁-C₆ et un alcényle en C₂-C₆,
   n est égal à 0 ou 1, préférentiellement égal à 1,
   Z représente un phényl, un pyrrole, un naphtalène, un aryle, un imidazole, un naphtyle ou un phénanthrène, préférentiellement un phényl, optionnellement substitué par au moins un radical choisi parmi un alkyle en C₁-C₃, un alcoxy en C₁-C₃ et un halogène, préférentiellement substitué par un halogène, ou deux halogènes,
   dans lequel
   quand C est un hydrogène, un méthyle ou un méthoxy, B est un groupe selon la formule (II), et inversement quand B est un hydrogène, un méthyle ou un méthoxy, C est un groupe selon la formule (II), et
   quand R1 est un hydrogène, un alkyle en C₁-C₃ ou un alcoxy en C₁-C₃, R2 est le groupe -Y-[W]n-Z, et inversement quand R2 est un hydrogène, un alkyle en C₁-C₃ ou un alcoxy en C₁-C₃, R1 est le groupe -Y-[W]n-Z,
   pour son utilisation dans une méthode de traitement d'une infection au VIH chez un sujet atteint du VIH et/ou pour son utilisation dans une méthode de traitement de la latence du VIH dans des cellules latentes infectées par le VIH chez un sujet atteint du VIH.

L'invention a plus particulièrement pour objet un composé selon la formule (I) capable d'activer la protéine Tat, ou un sel de celui-ci, pour son utilisation dans une méthode de traitement d'une infection au VIH chez un sujet atteint du VIH et/ou pour son utilisation dans une méthode de traitement de la latence du VIH dans des cellules latentes infectées par le VIH chez un sujet atteint du VIH.

L'invention a notamment pour objet un composé selon la formule (I), présentant une forte affinité pour le complexe de transcription Tat-TAR, et favorisant l'activité transcriptionnelle de cette protéine, ou un sel de celui-ci, pour son utilisation dans une méthode de traitement d'une infection au VIH chez un sujet atteint du VIH et/ou pour son utilisation dans une méthode de traitement de la latence du VIH dans des cellules latentes infectées par le VIH chez un sujet atteint du VIH.

L'invention a également pour objet l'utilisation d'un composé selon la formule (I) capable d'activer la protéine Tat, ou un sel de celui-ci, pour la préparation d'un médicament pour traiter une infection au VIH chez un sujet atteint du VIH et/ou pour la préparation d'un médicament pour traiter la latence du VIH dans des cellules latentes infectées par le VIH chez un sujet atteint du VIH.

L'invention a notamment pour objet l'utilisation d'un composé selon la formule (I), présentant une forte affinité pour le complexe de transcription Tat-TAR, et favorisant l'activité transcriptionnelle de cette protéine, ou un sel de celui-ci, pour la préparation d'un médicament pour traiter une infection au VIH chez un sujet atteint du VIH et/ou pour la préparation d'un médicament pour traiter la latence du VIH dans des cellules latentes infectées par le VIH chez un sujet atteint du VIH.

Dans un mode de réalisation, A est choisi parmi un benzimidazole, un imidazo[2,1-b]-1,3,4-thiadiazole, un benzodiazole, un benzothiazole, un benzoxazolone, une purine, une pyridine, un Oxazolone, une quinazoline et une quinoxaline, optionnellement substitué par au moins un halogène.

Dans un mode de réalisation, B et C représentent indépendamment l'un de l'autre, un radical choisi parmi un hydrogène et un groupe selon la formule (II).

Dans un mode de réalisation, B et C représentent indépendamment l'un de l'autre, un radical choisi parmi un C1-C6 alkoxy, préférentiellement un alkoxy en C₁-C₃, plus préférentiellement un méthoxy ou un éthoxy, et un groupe selon la formule (II).

Dans un mode de réalisation, B et C représentent indépendamment l'un de l'autre, un radical choisi parmi un C1-C6 alkyl, préférentiellement un méthyl ou un éthyl, et un groupe selon la formule (II).

Dans un mode de réalisation, le groupe -Y-[W]n-Z représente un naphtalènoxy, un pyrolméthoxy, un phenyloxy, ou un benzène, préférentiellement un benzyloxy, optionnellement substitué par un ou plusieurs radicaux choisis parmi un halogène et un C₁-C₃ alkyl.

Dans un mode de réalisation, C est un hydrogène, un méthyle ou un méthoxy, et B est un groupe selon la formule (II), dans lequel R1 est un méthoxy ou un hydrogène et R2 est un benzyloxy optionnellement substitué par un halogène, préférentiellement un chlore.

Dans un autre mode de réalisation, B est un hydrogène, un méthyle ou un méthoxy, et C est un groupe selon la formule (II), dans lequel R1 est un méthoxy ou un hydrogène et R2 est un benzyloxy optionnellement substitué par un halogène, préférentiellement un chlore.

Dans un mode de réalisation, C est un hydrogène, et B est un groupe selon la formule (II), dans lequel R1 est un hydrogène et R2 est un benzyloxy optionnellement substitué par au moins un halogène, préférentiellement un chlore, plus préférentiellement substitué par deux chlores.

Dans un autre mode de réalisation, B est un hydrogène, et C est un groupe selon la formule (II), dans lequel R1 est un hydrogène et R2 est un benzyloxy optionnellement substitué par au moins un halogène, préférentiellement un chlore, plus préférentiellement substitué par deux chlores.

Dans un mode de réalisation, C est un hydrogène, et B est un groupe selon la formule (II), dans lequel R1 est un méthoxy et R2 est un benzyloxy optionnellement substitué par au moins un halogène, préférentiellement un chlore, plus préférentiellement substitué par deux chlores.

Dans un autre mode de réalisation, B est un hydrogène, et C est un groupe selon la formule (II), dans lequel R1 est un méthoxy et R2 est un benzyloxy optionnellement substitué par au moins un halogène, préférentiellement un chlore, plus préférentiellement substitué par deux chlores.

Dans un mode de réalisation, C est un hydrogène, et B est un groupe selon la formule (II), dans lequel R1 est un éthyloxy et R2 est un benzyloxy optionnellement substitué par au moins un halogène, préférentiellement un chlore, plus préférentiellement substitué par deux chlores.

Dans un autre mode de réalisation, B est un hydrogène, et C est un groupe selon la formule (II), dans lequel R1 est un éthyloxy et R2 est un benzyloxy optionnellement substitué par au moins un halogène, préférentiellement un chlore, plus préférentiellement substitué par deux chlores.

Dans un mode de réalisation, C est un méthyle, et B est un groupe selon la formule (II), dans lequel R1 est un hydrogène et R2 est un benzyloxy optionnellement substitué par au moins un halogène, préférentiellement un chlore, plus préférentiellement substitué par deux chlores.

Dans un autre mode de réalisation, B est un méthyle, et C est un groupe selon la formule (II), dans lequel R1 est un hydrogène et R2 est un benzyloxy optionnellement substitué par au moins un halogène, préférentiellement un chlore, plus préférentiellement substitué par deux chlores.

Dans un mode de réalisation, C est un méthoxy, et B est un groupe selon la formule (II), dans lequel R1 est un hydrogène et R2 est un benzyloxy optionnellement substitué par au moins un halogène, préférentiellement un chlore, plus préférentiellement substitué par deux chlores.

Dans un autre mode de réalisation, B est un méthoxy, et C est un groupe selon la formule (II), dans lequel R1 est un hydrogène et R2 est un benzyloxy optionnellement substitué par au moins un halogène, préférentiellement un chlore, plus préférentiellement substitué par deux chlores.

Dans un mode de réalisation, C est un méthyl, et B est un groupe selon la formule (II), dans lequel R1 est un méthoxy et R2 est un benzyloxy optionnellement substitué par au moins un halogène, préférentiellement un chlore, plus préférentiellement substitué par deux chlores.

Dans un autre mode de réalisation, B est un méthyl, et C est un groupe selon la formule (II), dans lequel R1 est un méthoxy et R2 est un benzyloxy optionnellement substitué par au moins un halogène, préférentiellement un chlore, plus préférentiellement substitué par deux chlores.

Dans un mode particulier :
- A représente un benzimidazole éventuellement substitué par un halogène (de préférence un chlore), et
- B et C représentent indépendamment l'un de l'autre, un radical choisi parmi :
   un hydrogène,
   un C1-C6 alkyl,
   un C1-C6 alkoxy, et
   un groupe selon la formule (II), dans laquelle R₁ et R₂ représentent indépendamment l'un de l'autre,
   un radical choisi parmi :
      un hydrogène,
      un C1-C2 alkoxy,
      un groupe selon la formule -Y-[W]n-Z,
      dans lequel
      Y est un radical choisi parmi O, N, et C (de préférence O) ;
      W est un radical choisi parmi un méthyle (-CH₂-), un éthyle linéaire (-CH2-CH2-) et un éthényle (-CH=CH-),
      n est égal à 1,
      Z représente un phényl, un pyrrole ou un naphtalène, aryle, un imidazole, naphtyle, ou un phénanthrène (de préférence un phényle), optionnellement substitué par au moins un radical choisi parmi un C1-C3 alkyl, un C1-C3 alkoxy et un halogène.

Dans un autre mode particulier :
- A est un imidazo[2,1-b]-1,3,4-thiadiazole,
- B et C représentent indépendamment l'un de l'autre, un radical choisi parmi :
   un hydrogène,
   un C1-C6 alkoxy, et
   un groupe selon la formule (II) dans laquelle R₁ et R₂ représentent indépendamment l'un de l'autre, un radical choisi parmi
   un hydrogène,
   un C1-C2 alkoxy,
   un groupe selon la formule -Y-[W]n-Z,
   dans lequel
   Y est un radical choisi parmi O, N, et C (de préférence O);
   W est un alkyle en C1-C2,
   n est égal à 1 ;
   Z représente un phényl, un pyrrole ou un naphtalène, aryle, un imidazole, naphtyle, ou un phénanthrène (de préférence un phényl) optionnellement substitué par au moins un radical choisi parmi un C1-C3 alkyl, un C1-C3 alkoxy et un halogène,
   sous réserve que, lorsque R1 est un méthoxy, R2 est un groupe de formule (II) dans laquelle Z est un phényle substitué par un halogène (de préférence un chlore ou un fluor).

Dans un mode de réalisation particulier, A est benzoxazole, benzothiazole, pyridine, quinazoline, purine, imidazo[1,2a]pyrimidine, ou thiazolo[5,4-b]pyridine. Dans un tel mode particulier, on préfère que :
B et C représentent indépendamment l'un de l'autre, un radical choisi parmi :
un hydrogène,
un C1-C6 alkyl (de préférence, un méthyle),
un C1-C6 alkoxy (de préférence un méthoxy), et
un groupe selon la formule (II), dans laquelle R₁ et R₂ représentent indépendamment l'un de l'autre,
un radical choisi parmi :
un hydrogène,
un C1-C3 alkoxy (de préférence un méthoxy),
un groupe selon la formule -Y-[W]n-Z,
dans lequel
Y est un radical choisi parmi O, N, et C (de préférence O) ;
W est un radical choisi parmi un alkyle en C1-C2,
n est égal à 1 ;
Z représente un phényl, un pyrrole ou un naphtalène, aryle, un imidazole, naphtyle, ou un phénanthrène (de préférence un phényl) optionnellement substitué par au moins un radical choisi parmi un C1-C3 alkyl, un C1-C3 alkoxy et un halogène.

Dans un mode de réalisation particulier, le composé est choisi parmi le 4-(benzyloxy)-N-(5-{imidazo[2,1-b][1,3,4]thiadiazol-2-yl}-2-methoxyphenyl)benzamide, le N-[5-(1H-1,3-benzodiazol-2-yl)-2-methylphenyl]-4-[(4-chlorophenyl)methoxy]-3-methoxybenzamide, le N-[4-(1H-1,3-benzodiazol-2-yl)phenyl]-3-(benzyloxy)benzamide et le N-[3-(1H-1,3-benzodiazol-2-yl)phenyl]-4-[(4-chlorophenyl)methoxy]-3-methoxybenzamide, ou des sels de ceux-ci.

L'invention a également pour objet un composé selon la formule (I) ou un sel de celui-ci pour son utilisation dans une méthode de levée de la latence du VIH dans des cellules latentes infectées par le VIH ou pour la réactivation du VIH latent dans des cellules latentes infectées par le VIH chez un sujet atteint du VIH.

L'invention a également pour objet un composé selon la formule (I) capable d'activer la protéine Tat, ou un sel de celui-ci, pour son utilisation dans une méthode de levée de la latence du VIH dans des cellules latentes infectées par le VIH ou pour la réactivation du VIH latent dans des cellules latentes infectées par le VIH chez un sujet atteint du VIH.

L'invention a également pour objet un composé selon la formule (I) présentant une forte affinité pour le complexe de transcription Tat-TAR, et favorisant l'activité transcriptionnelle de cette protéine, ou un sel de celui-ci, pour son utilisation dans une méthode de levée de la latence du VIH dans des cellules latentes infectées par le VIH ou pour la réactivation du VIH latent dans des cellules latentes infectées par le VIH chez un sujet atteint du VIH.

L'invention a également pour objet l'utilisation d'un composé selon la formule (I) capable d'activer la protéine Tat, ou un sel de celui-ci, pour la préparation d'un médicament pour lever la latence du VIH dans des cellules latentes infectées par le VIH ou pour la préparation d'un médicament pour la réactivation du VIH latent dans des cellules latentes infectées par le VIH chez un sujet atteint du VIH.

L'invention a également pour objet l'utilisation d'un composé selon la formule (I) présentant une forte affinité pour le complexe de transcription Tat-TAR, et favorisant l'activité transcriptionnelle de cette protéine, ou un sel de celui-ci, pour la préparation d'un médicament pour lever la latence du VIH dans des cellules latentes infectées par le VIH ou pour la préparation d'un médicament pour la réactivation du VIH latent dans des cellules latentes infectées par le VIH chez un sujet atteint du VIH.

La description décrit également une méthode de traitement d'une infection au VIH chez un sujet atteint du VIH, qui n'appartient pas à l'invention revendiquée, comprenant une étape selon laquelle un ou plusieurs composés selon la formule (I) sont administrés audit patient de manière à réactiver le VIH dans des cellules latentes infectées par le VIH dudit patient.

La description décrit également une méthode de traitement d'une infection au VIH chez un sujet atteint du VIH, qui n'appartient pas à l'invention revendiquée, comprenant une étape selon laquelle un ou plusieurs composés selon la formule (I) et capable(s) d'activer la protéine Tat, sont administrés audit patient de manière à réactiver le VIH dans des cellules latentes infectées par le VIH dudit patient.

La description décrit également une méthode de traitement d'une infection au VIH chez un sujet atteint du VIH, qui n'appartient pas à l'invention revendiquée, comprenant une étape selon laquelle un ou plusieurs composés selon la formule (I) et présentant une forte affinité pour le complexe de transcription Tat-TAR, et favorisant l'activité transcriptionnelle de cette protéine, sont administrés audit patient de manière à réactiver le VIH dans des cellules latentes infectées par le VIH dudit patient.

Avantageusement, de telles méthodes de traitement comprennent également l'administration d'un traitement antiviral, notamment un traitement antirétroviral, afin notamment d'éliminer les cellules latentes qui ont été réactivées. L'administration audit sujet d'un ou plusieurs agents antiviraux peut se faire préalablement, simultanément et/ou séquentiellement à l'administration d'un ou plusieurs composés selon l'invention.

Selon l'invention, le composé est administré par voie parentérale, entérale ou cutanée, préférentiellement par voie parentérale, notamment par injection sous-cutanée ou intraveineuse, ou par voie entérale, notamment par voie orale.

L'invention a également pour objet une composition pharmaceutique comprenant au moins un composé de formule (I) et un support pharmaceutiquement acceptable.

La description décrit également une méthode de traitement d'un patient infecté par le VIH, qui n'appartient pas à l'invention revendiquée, ladite méthode comprenant les étapes selon lesquelles on administre audit patient une quantité efficace de la composition pharmaceutique comprenant au moins un composé de formule (I) et un support pharmaceutiquement acceptable, et on administre audit patient une quantité efficace d'un ou plusieurs agents antiviraux. Avantageusement le composé de formule (I) de ladite composition est avantageusement capable d'activer la protéine Tat et/ou présente une forte affinité pour le complexe de transcription Tat-TAR, et favorise l'activité transcriptionnelle de cette protéine.

L'invention a également pour objet un kit comprenant au moins un composé selon l'invention et au moins un agent antiviral et/ou au moins un autre agent de levée de latence (LRA), différent des composés selon l'invention. Avantageusement, le ou les LRAs supplémentaires ciblent une autre protéine que la protéine Tat. Notamment, le ou les LRAs supplémentaires peuvent cibler une protéine cellulaire. Avantageusement, ledit composé est conditionné pour être utilisé à une concentration comprise entre 0.03 µM et 15 µM, notamment une concentration comprise entre 3 µM et 10µM.

L'invention a également pour objet une utilisation in vitro / ex vivo d'un composé selon l'invention, pour la réactivation du VIH dans des cellules latentes infectées par le VIH.

### Brève description des figures

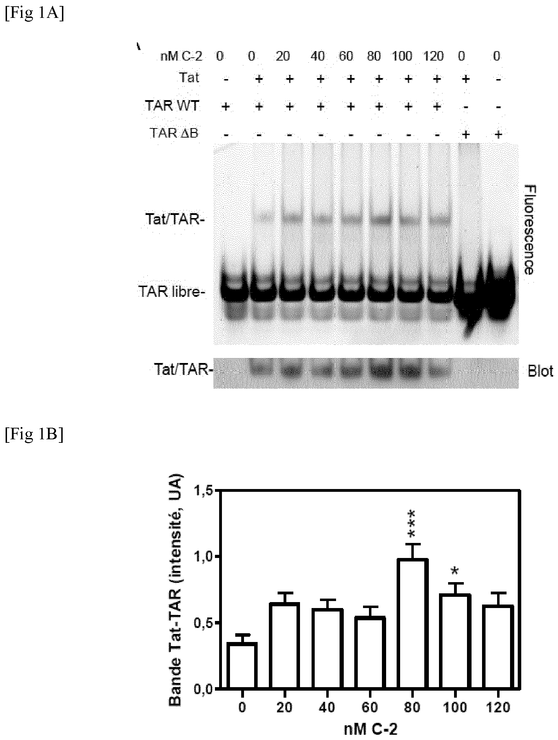

**[****Fig 1A****]** Gel retard montrant la migration de l'ARN TAR libre ou complexé à la protéine Tat (Tat/TAR) en présence de différentes concentrations (0 à 120 nM) du composé (2) (C-2). Tat (400 nM) a été incubée avec 200 nM de TAR-FAM (WT ou Δbulge) pendant 1,5 h en absence ou présence du composé (2), avant séparation sur un gel 0.5 X TBE 8% acrylamide, et acquisition de l'image en fluorescence. Les protéines du gel ont ensuite été transférées sur une membrane de nitrocellulose pour une révélation de Tat par Western blot.
**[****Fig 1B****]** Quantification de l'affinité du composé (2) (C-2) pour le complexe de transcription Tat-TAR. La bande de fluorescence Tat-TAR de gels dupliqués a été quantifiée et les résultats sont présentés dans le graphe (UA, unités arbitraires), n = 2. Moyennes +/- SEM. *, p<0.05 ; ***, p<0.001 comparé au contrôle sans le composé (2) (One-way ANOVA).

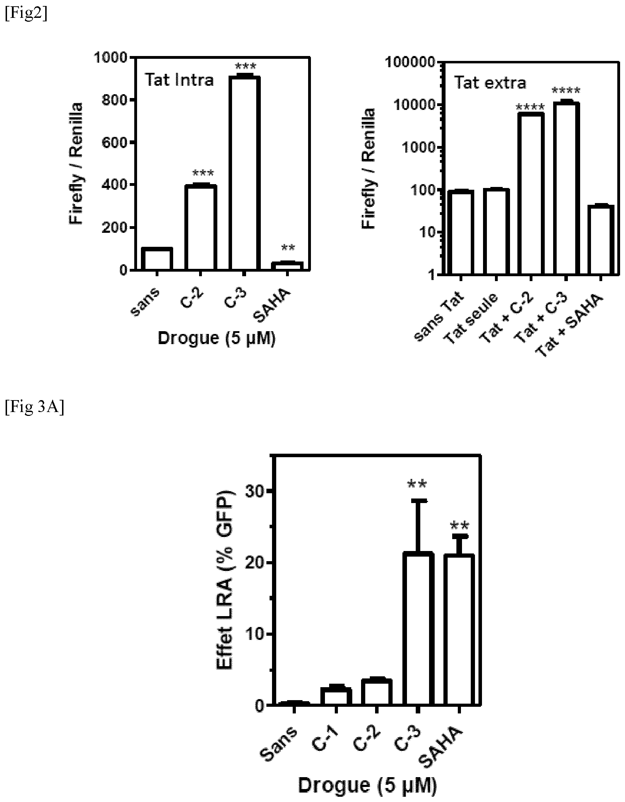

**[****Fig 2****]** Evaluation de l'activité transactivatrice de la protéine Tat de cellules HeLa en présence de 5 µM du composé (2) (C-2) ou du composé (3) (C-3). Des cellules HeLa ont été transfectées par des plasmides contenant les LTR-firefly et TK-renilla. Dans la condition Tat intra, Tat est co-transfectée. 18 h après transfection les composés (2) ou (3) ont été ajoutés (5µM), ainsi que 200 nM Tat pour la condition Tat extra. Les cellules sont lysées pour les tests luciférases 24 h après. Le rapport firefly / renilla obtenu dans les conditions contrôles a été fixé à 100. n = 2. Moyennes +/- SEM.**, p<0.01, ***, p< 0.001 et ****, p<0.0001 comparé au contrôle sans drogue (One-way ANOVA).
**[****Fig 3A****]** Activité LRA des composés (1), (2) (3) (C-1, C-2, C-3) et SAHA (inhibiteur des histones déacétylases) à 5µM sur cellules latentes J-Lat 9.2 (dérivées de Jurkats). Les J-Lat 9.2 possèdent un génome (unique) complet du VIH avec un gène Env non-fonctionnel, et une GFP à la place de Nef (Symons, J., et al., Retrovirology, 2017. 14(1): p. 2 ; Jordan, A., et al., The EMBO journal, 2003. 22(8): p. 1868-77). L'activation de ces virus peut être suivie par la production de GFP et donc par la détection de fluorescence verte dans les cellules, suivie par FACS. n = 3. Moyennes +/-SEM.**, p<0.01, comparé au contrôle sans drogue (One-way ANOVA).

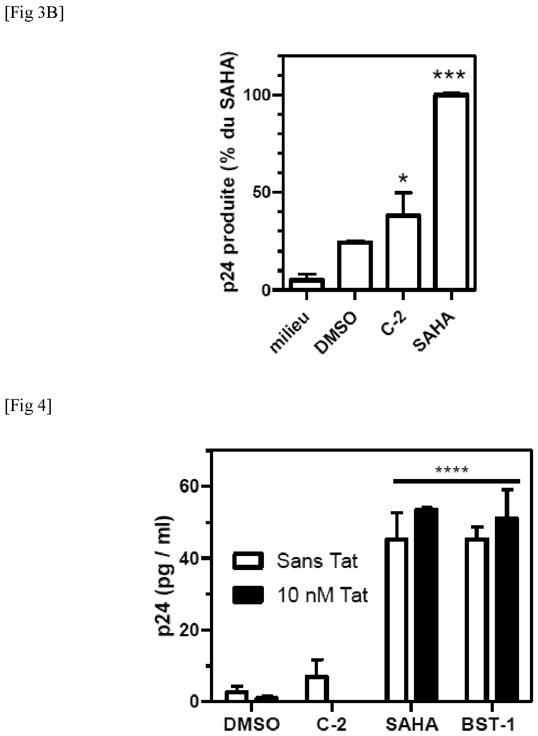

**[****Fig 3B****]** Activité LRA du composé (2) (C-2) et SAHA à 5 µM sur cellules latentes OM10.1 (promyéloblastes). Ces cellules latentes, produisent après induction, des virus infectieux qui sont dosés dans le surnageant par ELISA p24. n = 2. Moyennes +/- SEM.*, p<0.05 et ***, p< 0.001 comparé au contrôle milieu seul (One-way ANOVA).
**[****Fig 4****]** Evaluation de la spécificité du composé (2) (C-2) pour le complexe Tat-TAR : la production du VIH-1 dans la lignée cellulaire latente ACH-2 qui possède un TAR muté qui ne permet pas la transactivation par Tat a été mesurée, après mise en contact de cellules ACH-2 avec le composé (2) (5µM), SAHA (5µM) ou bryostatine-1 (BST-1 ; activateur de la protéine kinase C; 10 nM). n = 2. Moyennes +/- SEM. ****, p< 0.0001 comparé au contrôle solvant (One-way ANOVA).

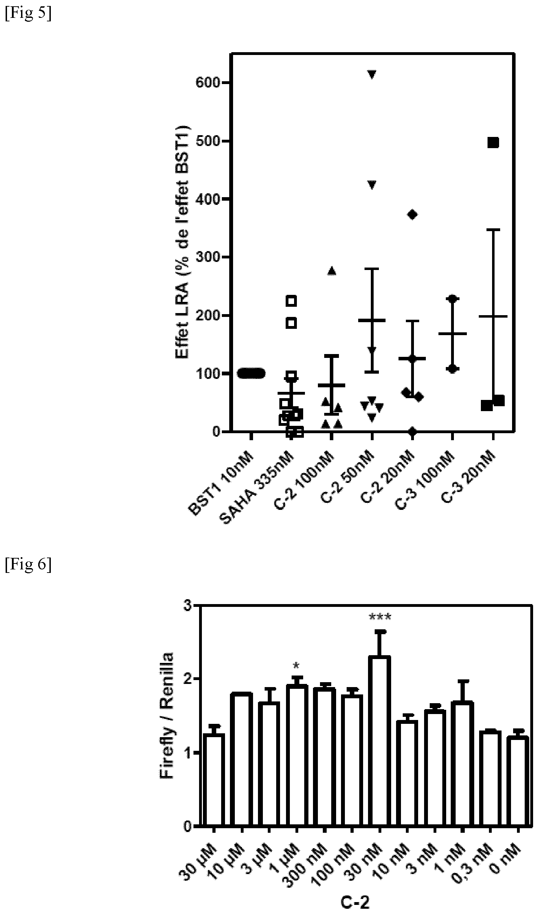

**[****Fig 5****]** Activité LRA *ex vivo* sur des cellules lymphocytaires T CD4⁺ quiescentes de patients VIH-1. Les cellules T-CD4 ont été isolées par sélection négative avant une élimination des cellules activées (CD25⁺, CD69⁺, HLA-DR⁺) pour obtenir les cellules quiescentes qui ont été traitées par les drogues pendant 18-20 h pour éviter les réinfections. L'activité LRA des composés (2) (C-2) et (3) (C-3) a été déterminée en suivant la protéine virale p24 dans le surnageant à l'aide d'un test ELISA commercial amélioré par l'utilisation d'un substrat luminescent. La limite de détection de la p24 a été de 0.2 pg/ml. La réponse à la BST-1 (10 nM) a été fixée à 100% pour permettre une comparaison de l'efficacité LRA entre les patients (1 point = 1 patient).
**[****Fig 6****]** Test de transactivation sur cellules T-CD4 primaires. Des cellules T-CD4 primaires ont été isolées de sang de donneurs sains, puis elles ont été transfectées par des plasmides contenant Tat, LTR-firefly et TK-renilla. 18 h après transfection différentes concentrations du composé (2) (C-2) ont été ajoutées, et les cellules lysées pour les tests luciférases 24 h après. n = 3. Moyennes +/-SEM. *, p<0.05 et ***, p< 0.001 comparé au contrôle sans C-2 (One-way ANOVA).

### Description détaillée

### Définitions

Par "halogène", on entend le fluor, le chlore, le brome ou l'iode.

Par "alkyle", on entend un groupe hydrocarboné aliphatique saturé, linéaire ou ramifié. Un « alkyle en C₁-C₃ » a de 1 à 3 atomes de carbone. Des exemples d'alkyle (ou alkyle en C₁-C₃) sont un méthyle, éthyle, ou propyle.

Par "hétéroaryle", on entend un groupe mono- ou polycyclique (principalement bi-cyclique) renfermant des doubles liaisons conjuguées, dont chaque cycle renferme de 3 à 6 chaînons et dont au moins un chaînon renferme un hétéroatome, notamment un groupe benzimidazole, un imidazo[2,1-b]-1,3,4-thiadiazole, un benzothiazole, un benzoxazolone, une purine Oxazolone, benzodiazole, quinazoline, quinoxaline, pyridine.

Par « alcoxy » ou « alkyloxy », on entend un alkyle tel que défini ci-dessus, rattaché au reste de la molécule via une liaison -O- (« -O-alkyle »). Un exemple d'alcoxy est notamment un groupe méthoxy ou éthoxy.

Par « alkylène », on entend un groupe divalent, hydrocarboné acyclique saturé, linéaire ou ramifié. Un « alkylène en C₁-C₆ » est un alkylène ayant 1 à 6 atomes de carbone, notamment, un méthylène, un éthylène, un propylène, un butylène, un pentylène ou un hexylène.

Par « alcényle », on entend un groupe hydrocarboné acyclique insaturé, linéaire ou ramifié, ayant au moins une double liaison carbone-carbone. Un « alcényle en C₂-C₆ » est un alcényle ayant 2 à 6 atomes de carbone, notamment un éthényle, un propényle, un butényle, un pentényle, ou un hexényle.

L'expression « substitué par au moins » signifie que le radical est substitué par un ou plusieurs groupes de la liste.

Dans le contexte de l'invention, les termes « sujet », « individu » ou « patient » sont interchangeables et se réfèrent à un animal, de préférence à un mammifère, encore plus préférablement à un humain, y compris un adulte, un enfant et un nouveau-né.

Les termes « traitement et « traiter » se réfèrent à une méthode pour soulager ou réduire une maladie et / ou les symptômes qui l'accompagnent.

Dans le contexte de l'invention, le terme "effet thérapeutique" se réfère à un effet induit par un ingrédient actif, ou une composition pharmaceutique selon l'invention, capable de prévenir ou de retarder l'apparition d'une maladie, ou de guérir ou d'atténuer les effets d'une maladie.

### Composés

En travaillant sur la possibilité de cibler des protéines virales pour réactiver des cellules latentes dans le cadre du traitement du VIH, les inventeurs ont identifié des composés capables de se fixer au complexe Tat-TAR et de stabiliser la protéine Tat dans un état conformationnel permettant de favoriser l'interaction avec son ARN cible. Les composés selon l'invention semblent agir en se fixant à la région centrale de la structure, à savoir le sillon principal autour du résidu tryptophane, et bloquer ainsi le complexe dans une conformation hyperactive.

Les composés selon l'invention répondent à la formule (I), 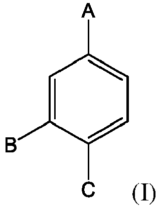 A représente un hétéroaryl optionnellement substitué par au moins un halogène.

Préférentiellement, A est choisi parmi un benzimidazole, un imidazo[2,1-b]-1,3,4-thiadiazole, un benzodiazole, un benzothiazole et un benzoxazolone, optionnellement substitué par au moins un halogène, préférentiellement par un chlore et/ou un brome. Par exemple, A est un benzimidazole optionnellement substitué par un chlore.

B et C représentent indépendamment l'un de l'autre, un radical choisi parmi un hydrogène, un alkyle en C₁-C₆, un alcoxy C₁-C₆, et un groupe selon la formule (II),

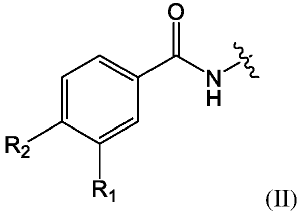

Dans un mode de réalisation, B est un hydrogène, un méthyle ou un méthoxy et C est un groupe selon la formule (II), dans laquelle R1 est un hydrogène ou un méthoxy et R2 est le groupe -Y-[W]n-Z, (ou inversement), dans lequel Y est O, n=1 et W est un méthyle et Z est un phényle, optionnellement substitué par un halogène, préférentiellement un chlore.

Ou inversement, C est un hydrogène, un méthyle ou un méthoxy et B est un groupe selon la formule (II), dans laquelle R1 est un hydrogène ou un méthoxy et R2 est le groupe -Y-[W]n-Z, (ou inversement), dans lequel Y est O, n=1 et W est un méthyle et Z est un phényle, optionnellement substitué par un halogène, préférentiellement un chlore.

Dans un mode de réalisation particulier, A est un benzimidazole ou un benzothiazole, C est un méthyle ou un hydrogène, et B est un groupe de formule (II) dans lequel R1 est un méthoxy, un éthoxy ou un hydrogène et R2 un benzyloxy, optionnellement substitué par un halogène et notamment par un chlore.

Ou inversement, dans lequel R1 un benzyloxy, optionnellement substitué par un halogène et notamment par un chlore, et R2 est un méthoxy, un éthoxy ou un hydrogène.

Dans un autre mode de réalisation, A est un benzimidazole ou un benzothiazole, B est un méthyle ou un hydrogène, et C est un groupe de formule (II) dans lequel R1 est un méthoxy, un éthoxy ou un hydrogène et R2 un benzyloxy, optionnellement substitué par un halogène et notamment par un chlore.

Ou inversement, dans lequel R1 un benzyloxy, optionnellement substitué par un halogène et notamment par un chlore, et R2 est un méthoxy, un éthoxy ou un hydrogène.

Dans un autre mode de réalisation, A est un imidazo[2,1-b]-1,3,4-thiadiazole, C est un méthoxy, et B est un groupe de formule (II) dans lequel R1 est un méthoxy, un éthoxy ou un hydrogène et R2 un benzyloxy, optionnellement substitué par un halogène et notamment par un chlore.

Ou inversement, dans lequel R1 un benzyloxy, optionnellement substitué par un halogène et notamment par un chlore, et R2 est un méthoxy, un éthoxy ou un hydrogène.

Dans un autre mode de réalisation, A est un imidazo[2,1-b]-1,3,4-thiadiazole, B est un méthoxy, et C est un groupe de formule (II) dans lequel R1 est un méthoxy, un éthoxy ou un hydrogène et R2 un benzyloxy, optionnellement substitué par un halogène et notamment par un chlore.

Ou inversement, dans lequel R1 un benzyloxy, optionnellement substitué par un halogène et notamment par un chlore, et R2 est un méthoxy, un éthoxy ou un hydrogène.

Dans un mode de réalisation, Y est un radical choisi parmi O, N et C.

Bien entendu, l'homme du métier comprendra que la valence des radicaux Y est respectée.

Il est ainsi compris par l'homme du métier que la définition "Y est N" est lue par l'homme du métier comme étant "Y est NH". De même, il est également compris par l'homme du métier que la définition "Y est C" est lue par l'homme du métier comme étant "C est CH2".

Dans un mode de réalisation, Y est un radical choisi parmi -O-, -NH- et -CH2-.

Avantageusement, W est un groupe divalent, c'est-à-dire qu'il est rattaché par une liaison à Y d'une part et par une autre liaison à Z d'autre part, chaque liaison impliquant le même carbone ou deux carbones différents dudit groupe divalent.

Par exemple, lorsque W est un groupe divalent « éthyle », le groupe -Y-(W)ₙ-Z inclut les deux groupes représentés ci-après :

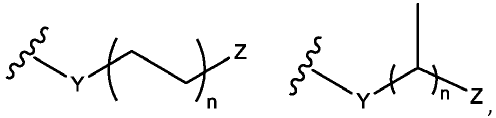

Où Y, Z et n sont tel que définis dans la présente demande.

Avantageusement, le composé selon la formule (I) est choisi parmi les composés (1) à (46) listés dans le tableau 1 ci-dessous. Plus avantageusement, le composé selon la formule (I) est choisi parmi les composés listés dans le tableau 1, capables d'activer la protéine Tat et/ou qui présentent une forte affinité pour le complexe de transcription Tat-TAR, et favorisent l'activité transcriptionnelle de cette protéine.[Table 1]

| | | |
|---|---|---|
| (1) | 4-(benzyloxy)-N-(5-{imidazo[2,1-b][1,3,4]thiadiazol-2-yl}-2-methoxyphenyl)benzamide | 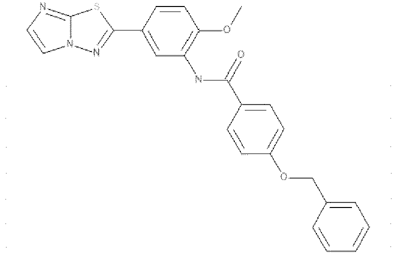 |
| (2) | N-[5-(1H-1,3-benzodiazol-2-yl)-2-methylphenyl]-4-[(4-chlorophenyl)methoxy]-3-methoxybenzamide | 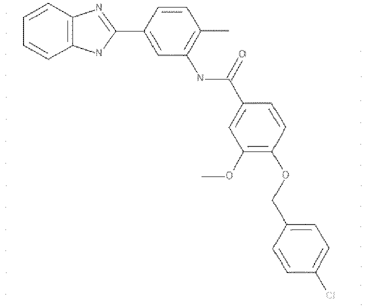 |
| (3) | N-[4-(1H-1,3-benzodiazol-2-yl)phenyl]-3-(benzyloxy)benzamide | 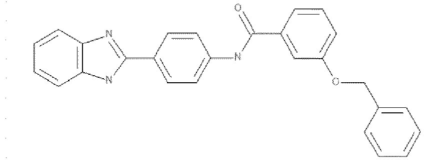 |
| (4) | N-[3-(1H-1,3-benzodiazol-2-yl)phenyl]-4-[(4-chlorophenyl)methoxy]-3-methoxybenzamide | 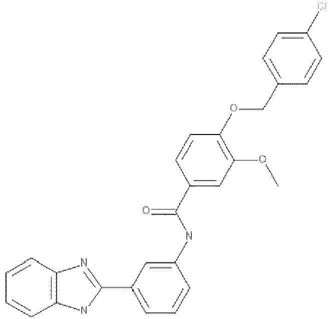 |
| (5) | N-[4-(1H-1,3-benzodiazol-2-yl)phenyl]-3-[(4-chlorophenyl)methoxy]benzamide | 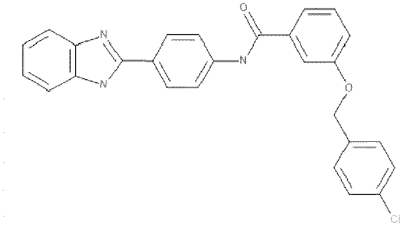 |
| (6) | N-[4-(1H-1,3-benzodiazol-2-yl)phenyl]-3-(2-phenylethoxy)benzamide | 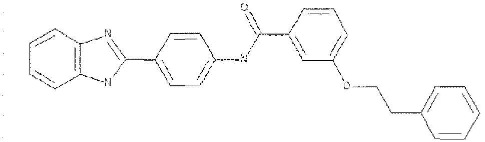 |
| (7) | 3-(benzyloxy)-N-[4-(7H-purin-8-yl)phenyl] benzamide | 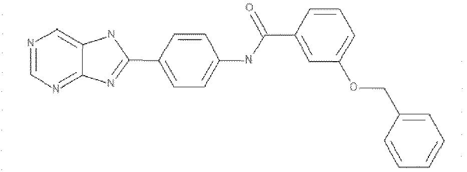 |
| (8) | 3-(benzyloxy)-N-[4-(5-chloro-1H-1,3-benzodiazol-2-yl)phenyl]benzamide | 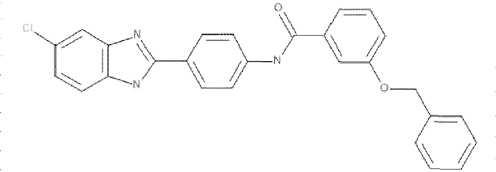 |
| (9) | N-[4-(1H-1,3-benzodiazol-2-yl)phenyl]-3-[(3,4-dichlorophenyl)methoxy]benzamide | 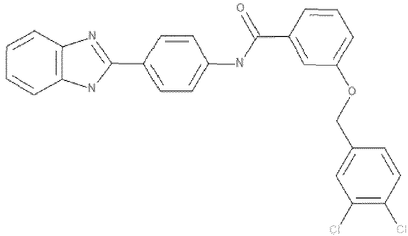 |
| (10) | N-[4-(1H-1,3-benzodiazol-2-yl)phenyl]-3-[(1R)-1-phenylethoxy]benzamide | 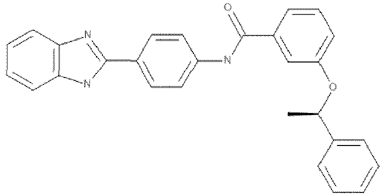 |
| (11) | N-[4-(1H-1,3-benzodiazol-2-yl)phenyl]-3-{[(1E)-2-phenylethenyl]oxy }benzamide | 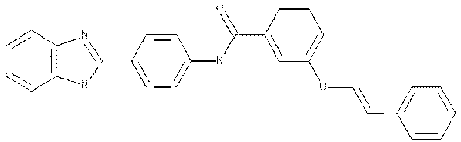 |
| (12) | N-[3-(1H-1,3-benzodiazol-2-yl)phenyl]-4-[(4-bromophenyl)methoxy]-3-methoxybenzamide | 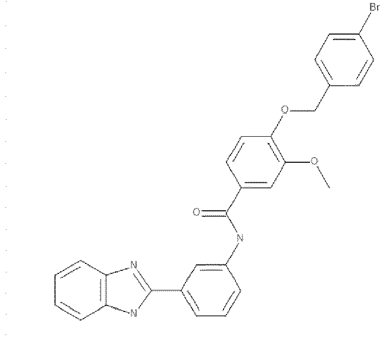 |
| (13) | N-[3-(1H-1,3-benzodiazol-2-yl)phenyl]-4-(benzyloxy)-3-methoxybenzamide | 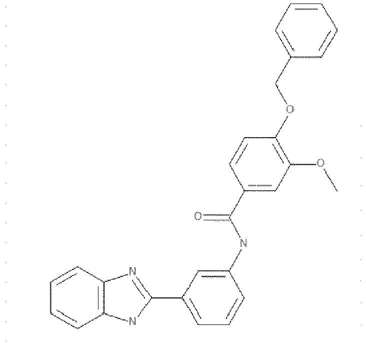 |
| (14) | N-[3-(1H-1,3-benzodiazol-2-yl)phenyl]-4-[(4-chlorophenyl)methoxy]-3-ethoxybenzamide | 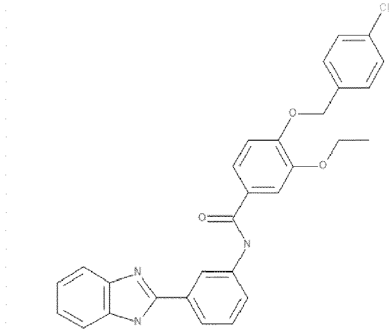 |
| (15) | N-[3-(1H-1,3-benzodiazol-2-yl)phenyl]-3-methoxy-4-[(1S)-1-phenylethoxy]benzamide | 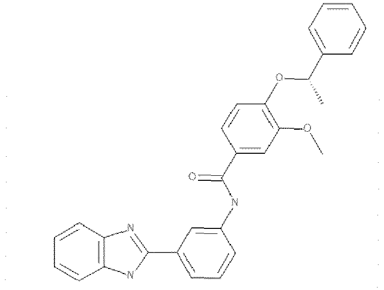 |
| (16) | N-[3-(1H-1,3-benzodiazol-2-yl)phenyl]-3-(benzyloxy)-4-[(4-chlorophenyl)methoxy]benzamide | 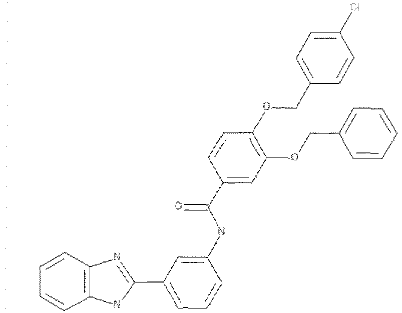 |
| (17) | N-[3-(1,3-benzothiazol-2-yl)phenyl]-4-[(4-chlorophenyl)methoxy]-3-methoxybenzamide | 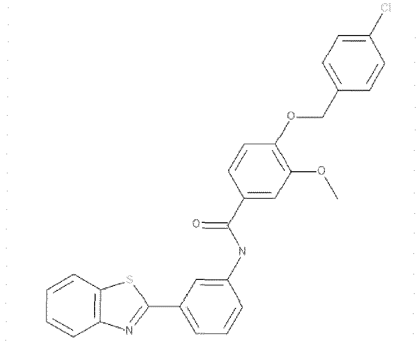 |
| (18) | N-[3-(1H-1,3-benzodiazol-2-yl)phenyl]-4-(benzylsulfanyl)-3-methoxybenzamide | 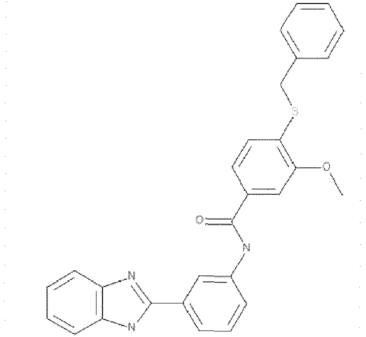 |
| (19) | 4-(benzyloxy)-N-(3-{imidazo[2,1-b][1,3,4]thiadiazol-2-yl}phenyl)-3-methoxybenzamide | 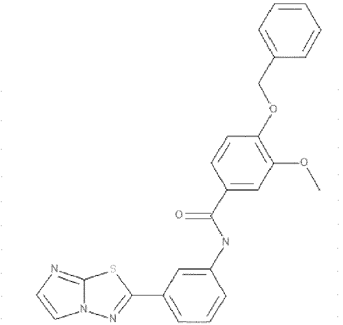 |
| (20) | 4-[(4-chlorophenyl)methoxy]-N-(3-{imidazo[2,1-b][1,3,4]thiadiazol-2-yl}phenyl)-3-methoxybenzamide | 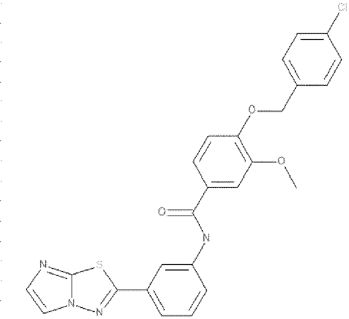 |
| (21) | N-(3-{imidazo[2,1-b][1,3,4]thiadiazol-2-yl}phenyl)-4-[(1S)-1-phenylethoxy]benzamide | 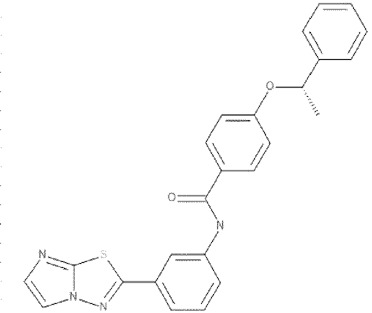 |
| (22) | 4-(benzyloxy)-3-ethoxy-N-(3-{imidazo[2,1-b][1,3,4]thiadiazol-2-yl}phenyl)benzamide | 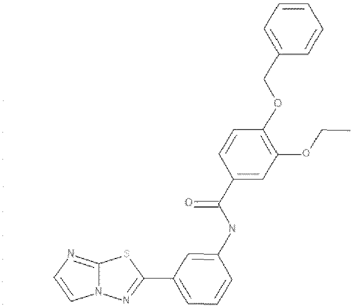 |
| (23) | 4-[(4-fluorophenyl)methoxy]-N-(3-{imidazo[2,1-b][1,3,4]thiadiazol-2-yl}phenyl)-3-methoxybenzamide | 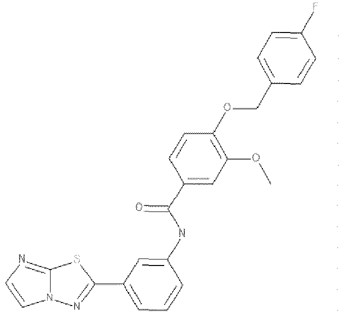 |
| (24) | 4-(benzyloxy)-N-(3-{[1,3]oxazolo[4,5-b]pyridin-2-yl}phenyl)benzamide | 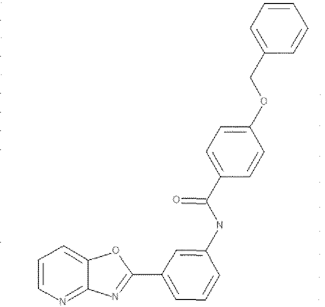 |
| (25) | N-[5-(1,3-benzothiazol-2-yl)-2-methylphenyl]-4-(benzyloxy)benzamide | 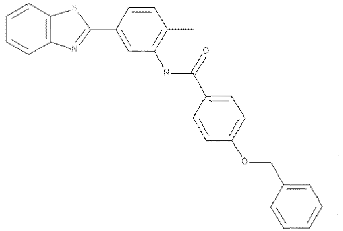 |
| (26) | N-[3-(1H-1,3-benzodiazol-2-yl)phenyl]-3-butoxybenzamide | 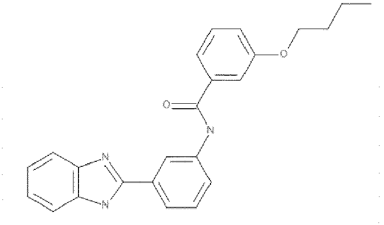 |
| (27) | N-[3-(1H-1,3-benzodiazol-2-yl)phenyl]-3-phenoxybenzamide | 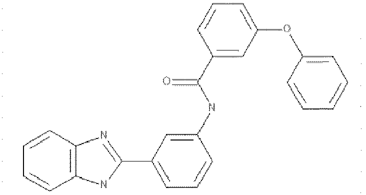 |
| (28) | N-[3-(1H-1,3-benzodiazol-2-yl)phenyl]-3-methoxy-4-[2-(morpholin-4-yl)-2-oxoethoxy]benzamide | 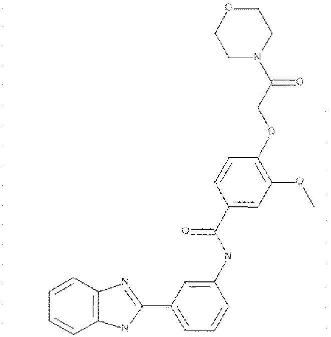 |
| (29) | N-[5-(1,3-benzoxazol-2-yl)-2-methoxyphenyl]-4-(benzyloxy)benzamide | 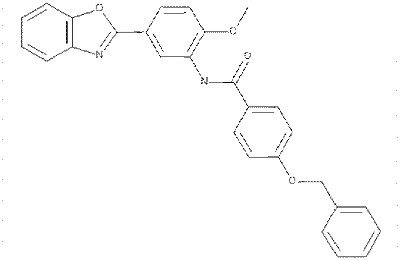 |
| (30) | N-[5-(1H-1,3-benzodiazol-2-yl)-2-methylphenyl]-4-(benzyloxy)benzamide | 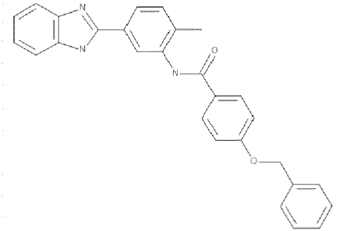 |
| (31) | N-[3-(1H-1,3-benzodiazol-2-yl)-4-chlorophenyl]-3,4,5-triethoxybenzamide | 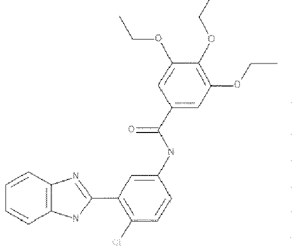 |
| (32) | 3-(benzyloxy)-N-(2-methyl-5-{3-methyl-[1,2,4]triazolo[3,4-b][1,3,4]thiadiazol-6-yl}phenyl)benzamide | 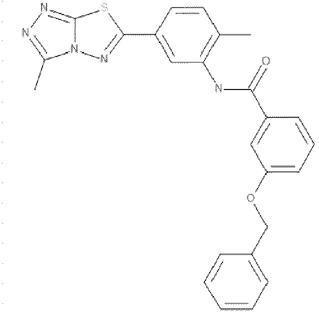 |
| (33) | N-[5-(1,3-benzoxazol-2-yl)-2-methoxyphenyl]-3-[(4-fluorophenyl)methoxy]benzamide | 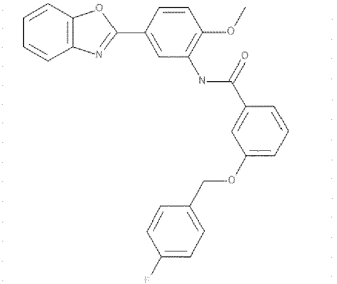 |
| (34) | N-[5-(1,3-benzoxazol-2-yl)-2-methoxyphenyl]-4-[(1R)-1-phenylethoxy]benzamide | 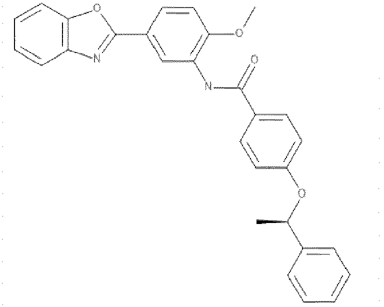 |
| (35) | N-[5-(1,3-benzoxazol-2-yl)-2-methoxyphenyl]-4-(benzyloxy)-3,5-difluorobenzamide | 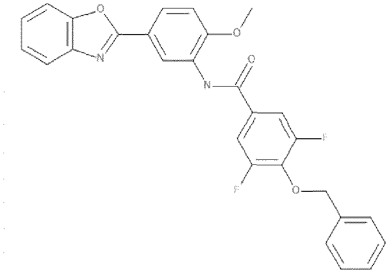 |
| (36) | N-[5-(1,3-benzoxazol-2-yl)-2-methoxyphenyl]-3-(benzyloxy)benzamide | 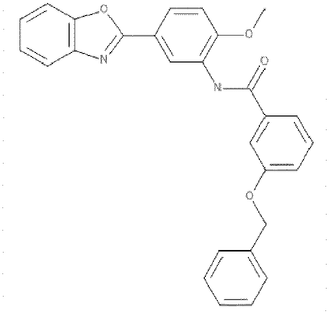 |
| (37) | 4-(benzyloxy)-N-[2-methoxy-5-(pyridin-3-yl)phenyl]benzamide | 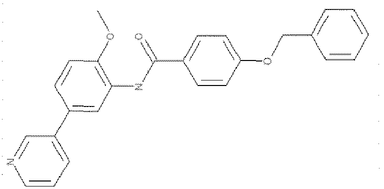 |
| (38) | 4-(benzyloxy)-N-[2-methoxy-5-(quinazolin-6-yl)phenyl]benzamide | 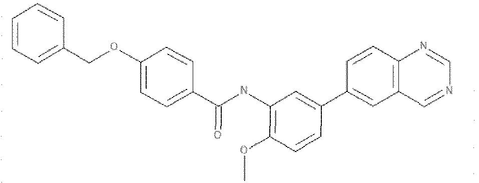 |
| (39) | 4-(benzyloxy)-N-[2-methoxy-5-(quinoxalin-6-yl)phenyl]benzamide | 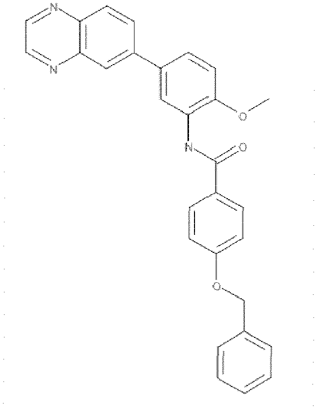 |
| (40) | 4-(benzyloxy)-N-(5-{imidazo[1,2-a]pyrimidin-6-yl}-2-methoxyphenyl)benzamide | 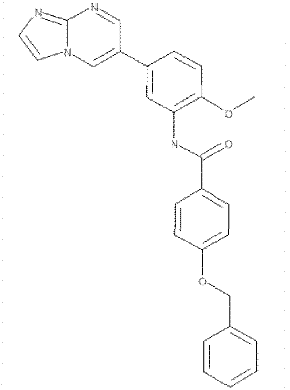 |
| (41) | 4-(benzyloxy)-N-(2-methoxy-5-{[1,3]thiazolo[5,4-b]pyridin-2-yl}phenyl)benzamide | 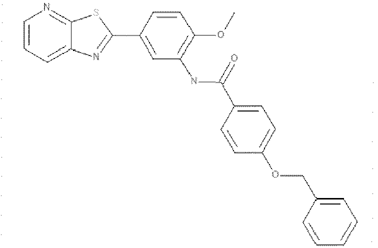 |
| (42) | N-[5-(1,3-benzoxazol-2-yl)-2-methoxyphenyl]-4-(benzyloxy)benzamide | 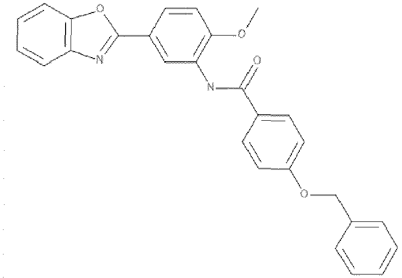 |
| (43) | N-[5-(1,3-benzoxazol-2-yl)-2-methylphenyl]-4-(benzyloxy)benzamide | 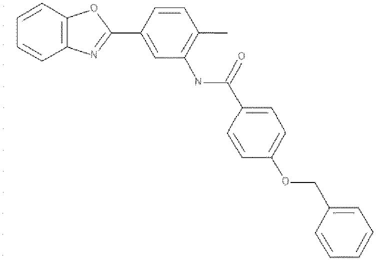 |
| (44) | N-[5-(1,3-benzoxazol-2-yl)-2-methoxyphenyl]-4-(benzyloxy)benzene-1-carbothioamide | 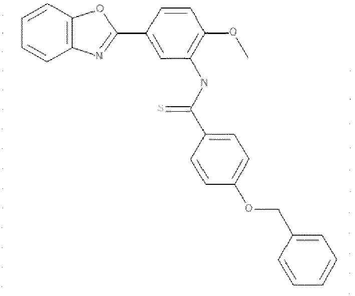 |
| (45) | 4-benzamido-N-[5-(1,3-benzoxazol-2-yl)-2-methoxyphenyl]benzamide | 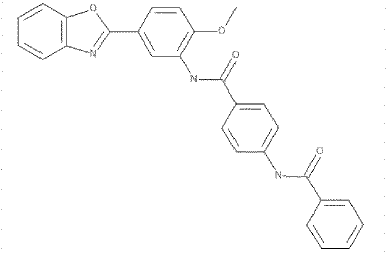 |
| (46) | 2-{3-[(1E)-2-[4-(benzyloxy)phenyl]diazen-1-yl]-4-methoxyphenyl}-1,3-benzoxazole | 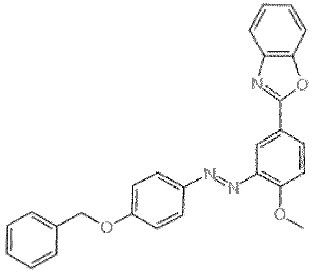 |

Dans un mode de réalisation particulier, le composé selon la formule (I) est choisi parmi les composés (1) à (9), (11) à (17), (20) à (23), (25), (29), (30), (33), (34), (36) à (38), (40) à (43) listés dans le tableau 1.

Dans un mode de réalisation, le composé répond à la formule (I) à la condition qu'il ne soit pas choisi parmi les composés (10), (18), (19), (24), (26), (27), (31), (32), (39) et (45) listés dans le tableau 1.

Dans un mode de réalisation, le composé répond à la formule (I) à la condition qu'il ne soit pas choisi parmi les composés (10), (18), (19), (24), (27), (32) et (39).

Dans un mode de réalisation, le composé est choisi parmi les composés répondant à la formule (I) listés dans le tableau 1, à la condition qu'il soit capable d'activer 1a protéine Tat.

Dans un mode de réalisation, le composé est choisi parmi les composés répondant à la formule (I) listés dans le tableau 1, à la condition qu'il présente une forte affinité pour le complexe de transcription Tat-TAR, et favorise l'activité transcriptionnelle de cette protéine.

L'invention vise également un sel pharmaceutiquement acceptable d'un composé de formule (I).

Il doit également être entendu que certains composés de formule (I) peuvent exister sous des formes solvatées aussi bien que non solvatées telles que, par exemple, des formes hydratées. Il doit être entendu que l'invention englobe toutes ces formes solvatées qui sont aptes à se fixer au complexe Tat-TAR.

### Composition pharmaceutique

La présente invention a également pour objet une composition pharmaceutique comprenant un ou plusieurs composés de formule (I) en tant que principe actif et un support pharmaceutiquement acceptable. Avantageusement, le ou les composés de la composition sont choisis parmi les composés de formule (I) capables d'activer la protéine Tat et/ou qui présentent une forte affinité pour le complexe de transcription Tat-TAR, et favorisent l'activité transcriptionnelle de cette protéine.

Dans le contexte de l'invention, les termes "principe actif", "ingrédient actif" et "ingrédient pharmaceutique actif" sont équivalents et se réfèrent à un composant d'une composition pharmaceutique ayant un effet thérapeutique.

Dans le contexte de l'invention, l'expression « excipient » ou « support pharmaceutiquement acceptable » fait référence à tout ingrédient à l'exception des ingrédients actifs qui est présent dans une composition pharmaceutique. Son ajout peut viser à conférer une consistance particulière ou d'autres propriétés physiques ou gustatives au produit final. Un excipient ou support pharmaceutiquement acceptable doit être dépourvu de toute interaction, notamment chimique, avec les principes actifs.

La composition pharmaceutique selon l'invention peut être formulée sous une forme destinée à une administration topique, entérale, orale, parentérale, intranasale, intraveineuse, intra-artérielle, intramusculaire, sous-cutanée ou intraoculaire et similaires. En particulier, la composition pharmaceutique selon l'invention peut être formulée pour une administration topique, entérale, orale, parentérale, intranasale, intraveineuse, intra-artérielle, intramusculaire, sous-cutanée ou intra-oculaire et similaires.

Pour une administration orale, la composition peut être formulée sous des formes posologiques orales classiques telles que des comprimés, des capsules, des poudres, des granulés et des préparations liquides telles que des sirops, des élixirs et des gouttes concentrées. Des supports ou diluants solides non toxiques peuvent être utilisés, qui comprennent, par exemple, des qualités pharmaceutiques de mannitol, lactose, amidon, stéarate de magnésium, saccharine sodique, talc, cellulose, glucose, saccharose, magnésium, carbonate et similaires. Pour les comprimés, des liants, qui sont des agents conférant des qualités cohésives aux matériaux en poudre, peuvent également être utilisés. Par exemple, l'amidon, la gélatine, les sucres tels que le lactose ou le dextrose, et les gommes naturelles ou synthétiques peuvent être utilisés comme liants. Des désintégrants peuvent également être utilisés dans les comprimés pour faciliter l'éclatement du comprimé. Les désintégrants comprennent les amidons, les argiles, les celluloses, les algines, les gommes et les polymères réticulés.

Pour l'administration transdermique, la composition peut être formulée sous forme de pommade, de crème ou de gel et des pénétrants ou détergents appropriés peuvent être utilisés pour faciliter la perméation, tels que le diméthylsulfoxyde, le diméthylacétamide et le diméthylformamide.

Pour l'administration transmuqueuse, des vaporisateurs nasaux, des suppositoires rectaux ou vaginaux peuvent être utilisés. Le composé actif peut être incorporé dans l'une quelconque des bases de suppositoire connues par des procédés connus dans l'art. Des exemples de telles bases comprennent le beurre de cacao, les polyéthylènes glycols (cires de carbowax), le monostéarate de polyéthylène sorbitane et leurs mélanges avec d'autres matériaux compatibles pour modifier le point de fusion ou la vitesse de dissolution.

Les compositions pharmaceutiques selon l'invention peuvent être formulées pour libérer le médicament actif sensiblement immédiatement après l'administration ou à tout moment ou période de temps prédéterminé après l'administration.

### Méthode de traitement et posologie

Les composés selon la présente invention sont des agents de levée de la latence aptes à réactiver le VIH dans des cellules latentes, infectées par le VIH.

La description décrit donc une méthode de traitement du VIH selon laquelle un ou plusieurs composés selon la formule (1), ou une composition selon l'invention sont administrés à un sujet atteint du VIH et présentant des cellules infectées latentes. Avantageusement, la méthode de traitement comprend également l'administration d'un agent antiviral.

La description décrit plus particulièrement une méthode de traitement de la latence du VIH dans des cellules infectées par le VIH chez un sujet atteint du VIH. Cette méthode de traitement de la latence peut avantageusement être intégrée dans une méthode plus générale de traitement du VIH selon laquelle, un ou plusieurs agents antiviraux sont également administrés audit sujet. Le but des composés selon l'invention étant de réactiver le VIH dans des cellules latentes infectées par le VIH, il sera le plus souvent nécessaire de compléter le traitement par les composés selon l'invention par un traitement par des agents antiviraux afin de neutraliser la production de VIH qui aura été réactivée. Notamment, le sujet peut être traité avec des agents antiviraux au moment du traitement de la latence au moyen d'un ou plusieurs composés selon l'invention ; et/ou avoir été traité avec des agents antiviraux préalablement au traitement de la latence au moyen d'un ou plusieurs composés selon l'invention ; et/ou être traité avec des agents antiviraux après le traitement de la latence au moyen d'un ou plusieurs composés selon l'invention.

Les composés selon l'invention peuvent également être utilisés in vitro / ex vivo pour réactiver le VIH dans des cellules latentes infectées par le VIH. Une telle utilisation peut notamment être utile in vitro pour traiter des cellules du réservoir, préalablement prélevées sur un patient infecté par le VIH. Une fois les cellules traitées in vitro, c'est-à-dire une fois les cellules latentes du réservoir réactivées in vitro, elles peuvent être réinjectées au patient.

La présente invention a également pour objet les composés et compositions pharmaceutiques décrits ci-dessus pour leur utilisation en tant que médicament, plus particulièrement pour leur utilisation dans le traitement d'une infection par le VIH, en particulier pour le traitement de la latence du VIH dans des cellules infectées par le VIH ; l'utilisation des composés et compositions pharmaceutiques décrits ci-dessus pour la fabrication d'un médicament destiné au traitement d'une infection par le VIH, en particulier pour le traitement de la latence du VIH dans des cellules infectées par le VIH .

Par quantité thérapeutiquement efficace est entendue une quantité permettant de réactiver le VIH dans des cellules infectées par le VIH et dans lesquelles le VIH est latent. Il est évident que la quantité à administrer peut être adaptée par l'homme du métier en fonction du sujet à traiter, de l'état d'avancement de la maladie, etc. En particulier, les doses et le schéma d'administration peuvent être fonction du stade et de la gravité de la maladie à traiter, ainsi que du poids, de l'âge et de la santé globale du sujet à traiter, ainsi que du jugement du médecin.

Dans un mode de réalisation particulier, la présente invention est relative à la composition pharmaceutique ou vaccinale ci-dessus décrite pour son utilisation dans le traitement d'une infection par le VIH, en particulier le VIH de type I, en combinaison avec un traitement antiviral ; l'utilisation de la composition pharmaceutique ou vaccinale ci-dessus décrite pour la fabrication d'un médicament ou vaccin destiné au traitement d'une infection par le VIH, en particulier le VIH de type I, en combinaison avec un traitement antiviral .

Le traitement antiviral des patients infectés par le VIH est bien connu de l'homme du métier. Notamment, différents antirétroviraux appartenant à plusieurs classes de médicaments différentes sont disponibles.

Notamment, les antirétroviraux peuvent être des inhibiteurs de transcriptase inverse, préférentiellement choisis parmi Abacavir, emtricitabine, lamivudine, ténofovir disoproxil fumarate, zidovudine, efavirenz, etravirine, nevirapine, rilpivirine, et même le doravirine ; des Inhibiteurs de protéase préférentiellement choisis parmi Atazanavir, darunavir, fosamprenavir, ritonavir, saquinavir, tipranavir, lopinavir ; des inhibiteurs de fusion, tel que enfuvirtide, des antagonistes de CCR5, tel que maraviroc, des inhibiteurs d'intégrase tels que Dolutégravir, raltégravir, elvitegravir, et même le cabotegravir ou bictegravir, des inhibiteurs post-attachement, notamment ibalizumab, des combinaisons, notamment dolutégravir, abacavir et lamivudine, ou zidovudine, abacavir et lamivudine, ou efavirenz, emtricitabine et ténofovir, ou efavirenz, lamivudine et ténofovir, ou bictefravir, emtricitabine et ténofovir, ou elvitegravir, cobicistat, emtricitabine et ténofovir, ou rilpivirine, emtricitabine et ténofovir, ou abacavir et lamivudine, ou abacavir, lamivudine et zidovudine, ou emtricitabine et ténofovir, ou lamivudine et zidovudine, ou lopinavir et ritonavir, ou atazanavir et cobicistat, ou darunavir et cobicistat, ou darunavir, cobicistat, emtricitabine et ténofovir, ou Dolutégravir et rilpivirine, ou Lamivudine et ténofovir.

Ainsi, lorsqu'il est fait référence ici à un traitement antiviral, il est notamment entendu tout médicament antirétroviral ou combinaison de ceux-ci, tels que décrits ci-dessus.

De préférence, le composé selon l'invention ou la composition pharmaceutique selon l'invention est administré par voie d'administration entérale ou parentérale. Lorsqu'il est administré par voie parentérale, le composé selon l'invention ou la composition pharmaceutique selon l'invention est de préférence administré par voie intraveineuse. Lorsqu'il est administré par voie entérale, le composé selon l'invention ou la composition pharmaceutique selon l'invention est de préférence administré par voie orale.

Le composé selon l'invention ou la composition pharmaceutique selon l'invention peut être administré en dose unique ou en doses multiples.

De préférence, le traitement est administré régulièrement, de préférence entre chaque jour et chaque mois, plus préférentiellement entre chaque jour et toutes les deux semaines, plus préférentiellement entre chaque jour et chaque semaine, encore plus préférentiellement le traitement est administré tous les jours. Dans un mode de réalisation particulier, le traitement est administré plusieurs fois par jour, de préférence 2 ou 3 fois par jour, encore plus préférentiellement 3 fois par jour.

La durée de traitement avec le composé selon l'invention ou la composition pharmaceutique selon l'invention est de préférence comprise entre 1 jour et 20 semaines, plus préférentiellement entre 5 jours et 10 semaines, encore plus préférentiellement entre 5 jours et 4 semaines, encore plus de préférence entre 5 jours et 2 semaines. Dans un mode de réalisation particulier, la durée du traitement est d'au moins 1 semaine.

La forme des compositions pharmaceutiques, la voie d'administration et la dose d'administration du composé selon l'invention, ou de la composition pharmaceutique selon l'invention peuvent être ajustées par l'homme du métier en fonction du type et de la sévérité de l'infection au VIH et au patient, en particulier son âge, son poids, son sexe et sa condition physique générale.

L'invention propose également un kit comprenant au moins un composé selon l'invention et au moins un agent antiviral, notamment au moins un agent antirétroviral et/ou au moins un autre agent de levée de latence (LRA). Avantageusement, le composé du kit est conditionné pour être utilisé à une concentration comprise entre 0.03 µM et 15 µM. Dans un mode de réalisation, le composé est conditionné pour être utilisé à une concentration comprise entre 3 µM et 15µM. Dans un autre mode de réalisation, le composé est conditionné pour être utilisé à une concentration comprise entre 0.03 µM et 0.1 µM.

### EXEMPLES

### Evaluation de l'affinité des composés selon l'invention pour la protéine Tat et le complexe de transcription Tat-TAR

La protéine Tat (86 résidus, isolat BH10) recombinante a été produite dans *E. coli* et purifiée sur héparine-Sepharose puis par HPLC comme décrit (Mol Biol Cell 15 (2004), 2347). Le composé (2) (MolPort) étant fluorescent (λex 320 nm ; λem 410nm), 1a technique de polarisation de fluorescence (Methods Appl. Fluoresc. (2016) 4(2)) et un fluorimètre PTI ont été utilisés pour l'étude de la fixation dudit composé (17 nM) sur la protéine Tat (1-60 µM) dans du tampon citrate (citrate 50 mM ; NaCl 150 mM; pH 7,2). Un Kd de l'ordre de 5 µM a été mesuré. Une valeur identique (~4 µM) a été obtenue en résonance plasmonique de surface (SPR ; Biacore) en passant le composé (2) dans du tampon citrate sur de la Tat immobilisée sur une puce CM5. Ces mesures montrent que le composé (2) a une affinité assez modeste pour Tat, et surtout cette valeur de Kd était bien supérieure à la concentration suffisante à l'effet LRA du composé (2) ex vivo qui est de 50-100 nM (voir plus bas).

L'affinité du composé (2) pour le complexe Tat-TAR a été étudiée par gel retard. Pour cela, de l'ARN cible (TAR 30 bases ; 200 nM ; WT ou Δ bulge, *i.e.* dépourvu de l'excroissance permettant la fixation de Tat) marqué à la fluorescéine (Sigma) a été mélangé avec 400 nM Tat +/- composé (2) dans 30 mM Tris (pH 7,5), 100 mM KCl, 2 mM dithiothreitol (DTT), 1 U RNAsin (Promega) /µl, 0,03% NP40, 10% glycérol, 5 µg polyIC (Sigma) / ml, et de l'eau sans RNAse. Après incubation 1,5 h à 25°C, le mélange est déposé sur un gel d'acrylamide à 8% dans du tris-borate-EDTA (TBE ; migration 1h20 à 4°C) (Figure 1A). La sonde libre migre au front et elle est retardée si Tat se fixe dessus. La fluorescence du gel est d'abord visualisée avec un Chemidoc (Biorad) pour détecter la fluorescéine (*i.e.* TAR). Les protéines du gel sont ensuite transférées sur une membrane de nitrocellulose pour détecter Tat par Western blot en utilisant un anticorps monoclonal (souris) anti-Tat (sc-65912) puis un anticorps de lapin anti-souris-peroxydase (Jackson Immunoresearch). La membrane est révélée avec de l'ECL select (GE Healthcare) visualisé avec le Chemidoc. La présence du composé (2) favorise l'interaction Tat-TAR. Comme cela est représenté à la figure 1B, le composé (2) a une affinité très forte pour le complexe de transcription Tat-TAR (~80 nM).

### Evaluation de la cytotoxicité des composés selon l'invention

La cytotoxicité du composé (2) et des autres molécules a été évaluée sur des cellules primaires T CD4 humaines (cellules aptes à devenir latentes). Les cellules ont été purifiées à partir de sang de donneur sain (convention avec l'EFS) par gradient Ficoll-Hypaque puis sélection négative des T-CD4 (Miltenyi Biotec 130-096-533). Elles ont ensuite été activées par la phytohémagglutinine (1µg/ml) puis par l'interleukine-2 (50 U/ml) pendant 5-7 jours (Nature comms 2018 9(1) 2251). Elles sont ensuite incubées (1 million / ml) 24 h avec le composé (2) (1 nM-100µM) en plaques 96 puits. La viabilité cellulaire est testée en utilisant le réactif CellTiter-Blue (Promega) comme recommandé par le fabriquant avant lecture de DO à l'aide d'un Tecan SPARK 10M. Les concentrations > 30 µM se sont révélées légèrement cytotoxiques. Des résultats similaires ont été obtenus en utilisant des lignées cellulaires humaines HEK (293/T17 de l'ATCC) ou HeLa (CCL2 de l'ATCC). Aucune toxicité significative n'a été observée pour des concentrations <10 µM, quel que soit le composé.

### Evaluation de l'effet des composés selon l'invention sur la transactivation par la protéine Tat

L'hypothèse de départ était que comme les cellules latentes expriment un certain niveau de protéines virales, si la transactivation par Tat est activée, les cellules infectées de façon latente par le VIH-1 pourront être réactivées pour être ensuite ciblées par l'ART et/ou lysées par les cellules T cytotoxiques.

Pour ce test, l'effet des molécules sur la transactivation par la protéine Tat extracellulaire ou intracellulaire a été suivi par l'activité de deux luciférases. D'une part la luciférase Firefly, qui est sous le contrôle d'un promoteur viral LTR dépendant de la protéine Tat, et d'autre part la luciférase Renilla (contrôle interne), sous le contrôle d'un promoteur cellulaire Thymidine kinase indépendant de la protéine Tat. La transactivation est exprimée par le rapport Firefly / Renilla. Tous les plasmides et la procédure ont été décrits (Mol Biol Cell 15 (2004), 2347).

Cette série d'expérience a été réalisée dans la lignée HeLa. Les cellules ont été transfectées avec le PEImax (Nature Comms 9 (2018) 2251). Les drogues et Tat (si Tat extra) ont été ajoutées 18h après transfection comme indiqué, et laissées 24h avant de lyser les cellules. Les luciférases sont ensuite dosées en utilisant le kit Dual-Glo de Promega et un lecteur de plaque Tecan SPARK 10M. La Tat extracellulaire a la propriété de pouvoir entrer dans les cellules. Il en résulte une concentration intracellulaire de Tat très faible (Mol Biol Cell 15 (2004), 2347), très probablement voisine de celle présente dans les cellules latentes.

Ainsi, la Tat recombinante seule ajoutée sur les cellules n'a pas d'effet significatif comparé au contrôle. Par contre, avec les composés (2) et (3) à 5 µM, la transactivation par Tat extracellulaire est très forte 60 fois (C-2) et 108 fois (C-3) celle du contrôle (Fig.2 ; Tat extra).

Les mêmes effets des composés (2) et (3) sur la transactivation par Tat ont été notés lorsqu'ils ont été testés avec la protéine Tat intracellulaire. Dans ce cas, Tat est transfectée, ce qui induit une forte concentration de Tat intracellulaire. En présence de 5 µM des composés, la transactivation par la protéine Tat intracellulaire a été multipliée 3.9 fois (C-2) et 9 fois (C-3), en comparaison avec le contrôle négatif (Fig.2 ; Tat intra). Les composés (2) et (3) agissent donc directement sur l'activité transcriptionnelle de Tat et pas en favorisant son entrée dans la cellule.

### Evaluation de l'activité LRA des composés selon l'invention sur lignées de cellules latentes

Pour tester l'effet LRA des molécules, des cellules JLat9.2 ont d'abord été utilisées. Cette lignée de cellules latentes (source : NIH AIDS Reagents Program, NARP) dérivées de cellules Jurkat contiennent un génome du VIH avec un gène Env inactivé et une GFP à la place de Nef (Retrovirology, 14 (2017) 2). La levée de latence s'accompagne de l'émission de fluorescence de la GFP. Les cellules ont été traitées par les molécules à 5µM pendant 24 h avant analyse des cellules avec un FACScalibur pour déterminer le % de cellules émettant de la fluorescence. On voit à la Figure 3A que le composé (2) à 5 µM a une activité LRA significative, de l'ordre de 20-30 % de celle du SAHA. De même, le composé (3) confirme les résultats en transactivation avec une activité LRA voisine de celle du SAHA. Le composé (1) présente également une activité significative.

Les résultats pour le composé (2) ont été confirmés en utilisant une autre lignée cellulaire latente, les OM10.1 (promyéloblastes;source NARP; PLoS Pathog. 11 (2015) 1) qui elles produisent un VIH infectieux après induction. Il a été dosé par l'antigène viral p24 (protéine de capside du VIH-1) dans le surnageant en utilisant un kit ELISA (Innotest). Sur ce modèle de latence plus physiologique le composé (2) montre une meilleure activité, de l'ordre de 40 % de celle du SAHA (Figure 3B).

### Spécificité des composés selon l'invention

La spécificité des composés a été évaluée en utilisant la lignée cellulaire latente ACH-2 (Source NARP) qui possède un génome du VIH avec un TAR muté (Retrovirology, 14 (2017) 2) qui ne permet pas 1a transactivation par Tat (J. Virol. 68 (1994) 1993). Une fois induites ces cellules produisent des virions infectieux. Pour ces tests, l'antigène viral p24 dans le surnageant des cellules a été dosé avec le kit ELISA p24 Innotest.

On voit (Fig.4) que le composé (2) (5 µM) est incapable d'induire 1a production du VIH-1 dans ces cellules alors que d'autres LRAs (SAHA et BST-1) le peuvent. L'activité LRA du composé (2) passe donc bien par le complexe de transcription viral Tat-TAR.

### Activité LRA des composés selon l'invention sur les cellules latentes de patients VIH-1

L'activité LRA *ex vivo* des composés selon l'invention a été testée sur des cellules lymphocytaires T CD4⁺ latentes de patients VIH-1. Tous les 8 patients étaient sous traitement ART depuis au moins 6 mois et leur charge virale était indétectable. Des prélèvements sanguins (20 ml) ont été réalisés (convention avec le CHU de Montpellier, Resp: Dr Edouard Tuaillon). Après une purification des globules blancs sur Ficoll-Hypaque, une sélection négative des T CD4⁺, une élimination des T-CD4 activés (CD25⁺, CD69⁺, HLA-DR⁺) (kits Milenyi), les cellules quiescentes ont été traitées par les composés pendant 18-20 h pour éviter les réinfections. Un dosage de la p24 a alors été effectué en utilisant un réactif luminescent (Luminata forte, Millipore) à la fin de l'Innotest. La sensibilité du test ainsi modifié est de ~0,2 pg p24 / ml. La réponse à la BST-1 variait selon les patients de 0,2 à 80 pg/ml.

On voit (Figure 5) que les composés (2) et (3) à 50-100 nM sont au moins aussi efficaces que la BST-1 qui est considérée comme le meilleur LRA disponible actuellement.

### Doses efficaces du composé (2)

Il a été montré que le composé (2) stabilise le complexe de transcription pour des concentrations de l'ordre de 50-100 nM, concentrations efficaces qui sont les mêmes que celles pour les tests LRA sur cellules primaires. Or, dans tous les tests sur des lignées cellulaires de laboratoire (HeLa et JLat), qui sont des lignées cancéreuses immortalisées, les concentrations efficaces sont de l'ordre de 5 µM. En fait, ces lignées surexpriment les transporteurs de drogues qui peuvent expulser (et/ou capter moins efficacement) différents types de molécules, ce qui pourrait expliquer la nécessité d'utiliser des concentrations plus fortes.

Pour vérifier ce point, un test de transactivation a été réalisé avec des cellules T-CD4 primaires. Le résultat montre que la concentration du composé (2) la plus efficace pour activer la transactivation par Tat est 30 nM (Fig.6), la dose efficace étant très proche de celle observée sur les primaires latentes (50 nM ; Fig.5) et celle optimisant la stabilité du complexe Tat-TAR (80 nM ; Fig.1). La nécessité d'utiliser des doses 100 fois plus fortes sur les cellules cancéreuses (*i.e.* 5-10 µM) est due au processus de cancérisation de ces cellules.

### Conclusion

Les composés selon l'invention sont des activateurs efficaces et sûrs de la protéine Tat du VIH-1. Ces composés sont aussi efficaces *ex vivo* sur les cellules de patients VIH⁺ que les meilleures LRAs disponibles actuellement qui ciblent des protéines cellulaires et montrent de nombreux effets secondaires. Les composés selon l'invention sont spécifiques de Tat et plus précisément interagissent avec une forte affinité avec le complexe de transcription viral Tat-TAR. Une concentration de 30-100 nM est suffisante pour assurer un effet maximal de ces composés *ex vivo.*

## Revendications

1. Composé selon la formule (1), ou un sel de celui-ci, 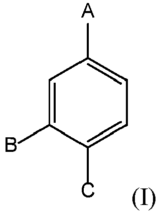 dans lequel
A représente un hétéroaryl optionnellement substitué par au moins un halogène, et
B et C représentent indépendamment l'un de l'autre, un radical choisi parmi :
un hydrogène,
un C1-C6 alkyl,
un C1-C6 alkoxy, et
un groupe selon la formule (II), 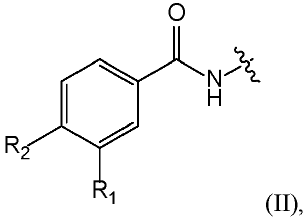
dans laquelle R₁ et R₂ représentent indépendamment l'un de l'autre, un radical choisi parmi un hydrogène,
un C1-C3 alkyl,
un C1-C3 alkoxy,
un groupe selon la formule -Y-[W]n-Z,
dans lequel
Y est un radical choisi parmi O, N, S et C ;
W est un radical choisi parmi un alkyle en C1-C6, un alkylène en C1-C6 et alcényle en C2-C6 ;
n est égal à 0 ou 1 ;
Z représente un phényl, un pyrrole ou un naphtalène, aryle, un imidazole, naphtyle, ou un phénanthrène optionnellement substitué par au moins un radical choisi parmi un C1-C3 alkyl, un C1-C3 alkoxy et un halogène
dans lequel
quand C est un hydrogène, un méthyl ou un méthoxy, B est un groupe selon la formule (II), et inversement quand B est un hydrogène, un méthyl ou un méthoxy, C est un groupe selon la formule (II), et
quand R1 est un hydrogène, un C1-C3 alkyl ou un C1-C3 alkoxy, R2 est le groupe -Y-[W]n-Z, et inversement quand R2 est un hydrogène, un C1-C3 alkyl ou un C1-C3 alkoxy, R1 est le groupe -Y-[W]n-Z,
pour son utilisation dans une méthode de traitement d'une infection au VIH chez un sujet atteint du VIH et/ou pour son utilisation dans une méthode de traitement de la latence du VIH dans des cellules latentes infectées par le VIH chez un sujet atteint du VIH.

2. Composé pour son utilisation selon la revendication 1, dans lequel A est choisi parmi un benzimidazole, un imidazo[2,1-b]-1,3,4-thiadiazole, un benzothiazole, un benzoxazolone, une purine Oxazolone, benzodiazole, quinazoline, quinoxaline, oxazolone pyridine, optionnellement substitué par au moins un halogène.

3. Composé pour son utilisation selon la revendication 1 ou 2, dans lequel A est un benzimidazole optionnellement substitué par au moins un halogène, préférentiellement un chlore et/ou un brome.

4. Composé pour son utilisation selon l'une des revendications précédentes, dans lequel le groupe -Y-[W]n-Z représente un naphtalenoxy, un pyrolmethoxy, un benzyloxy, un benzène ou un phényloxy, optionnellement substitué par un ou plusieurs radicaux choisis parmi un halogène et un C1-C3 alkyl.

5. Composé pour son utilisation selon l'une des revendications 1 à 4, dans lequel R1 est un méthoxy et R2 est un benzyloxy optionnellement substitué par un halogène, préférentiellement un chlore.

6. Composé pour son utilisation selon l'une des revendications 1 à 4, dans lequel R1 est un hydrogène et R2 est un benzyloxy optionnellement substitué par un halogène, préférentiellement un chlore.

7. Composé pour son utilisation selon l'une des revendications précédente, ledit composé étant choisi parmi le 4-(benzyloxy)-N-(5-{imidazo[2,1-b][1,3,4]thiadiazol-2-yl}-2-methoxyphenyl)benzamide, le N-[5-(1H-1,3-benzodiazol-2-yl)-2-methylphenyl]-4-[(4-chlorophenyl)methoxy]-3-methoxybenzamide, le N-[4-(1H-1,3-benzodiazol-2-yl)phenyl]-3-(benzyloxy)benzamide et le N-[3-(1H-1,3-benzodiazol-2-yl)phenyl]-4-[(4-chlorophenyl)methoxy]-3-methoxybenzamide, ou des sels de ceux-ci.

8. Composé pour son utilisation selon l'une des revendications précédentes, dans laquelle ladite méthode comprend l'administration du composé selon la formule (I) pour la réactivation du VIH dans des cellules latentes infectées par le VIH dudit sujet.

9. Composé pour son utilisation selon l'une des revendications précédentes, dans lequel ledit composé est administré par voie parentérale, entérale ou cutanée.

10. Composé pour son utilisation selon l'une des revendications précédentes, dans lequel la méthode comprend en outre l'administration audit sujet d'un agent antiviral.

11. Composition pharmaceutique comprenant le composé selon l'une des revendications 1 à 7 et un support pharmaceutiquement acceptable.

12. Kit comprenant au moins un composé selon l'une des revendications 1 à 7 et au moins un agent antiviral et/ou au moins un autre agent de levée de latence (LRA), ledit composé étant avantageusement conditionné pour être utilisé à une concentration comprise entre 0.03 µM et 15 µM, notamment entre 3 µM et 10µM ou entre 0.03 µM et 0.1 µM.

13. Utilisation in vitro / ex vivo d'un composé selon l'une des revendications 1 à 7 pour la réactivation du VIH dans des cellules latentes infectées par le VIH.

## Patentansprüche

1. Verbindung gemäß Formel (1) oder ein Salz davon, 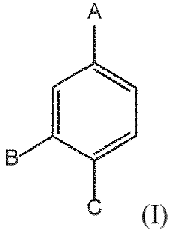 wobei
A ein Heteroaryl darstellt, das gegebenenfalls durch mindestens ein Halogen substituiert ist, und
B und C unabhängig voneinander einen Rest darstellen, ausgewählt aus:
einem Wasserstoff,
einem C1-C6 Alkyl,
einem C1-C6 Alkoxy, und
einer Gruppe gemäß Formel (II), 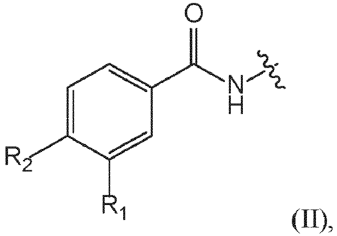
wobei R₁ und R₂ unabhängig voneinander einen Rest darstellen, ausgewählt aus
einem Wasserstoff,
einem C1-C3 Alkyl,
einem C1-C3 Alkoxy,
einer Gruppe gemäß Formel -Y-[W]n-Z,
wobei
Y ein Rest ist, ausgewählt aus O, N, S und C;
W ein Rest ist, ausgewählt aus einem C1-C6-Alkyl, einem C1-C6-Alkylen und einem C2-C6-Alkenyl; n gleich 0 oder 1 ist;
Z ein Phenyl, ein Pyrrol oder ein Naphthalin, Aryl, ein Imidazol, Naphthyl oder ein Phenanthren darstellt, gegebenenfalls substituiert durch mindestens einen Rest ausgewählt aus einem C1-C3-Alkyl, einem C1-C3-Alkoxy und einem Halogen
wobei
wenn C Wasserstoff, Methyl oder Methoxy ist, B eine Gruppe gemäß Formel (II) ist, und umgekehrt, wenn B Wasserstoff, Methyl oder Methoxy ist, C eine Gruppe gemäß Formel (II) ist, und
wenn R1 ein Wasserstoff, ein C1-C3-Alkyl oder ein C1-C3-Alkoxy ist, R2 die Gruppe -Y-[W]n-Z ist, und umgekehrt, wenn R2 ein Wasserstoff, ein C1-C3-Alkyl oder ein C1-C3-Alkoxy ist, R1 die Gruppe -Y-[W]n-Z ist,
zur Verwendung in einem Verfahren zur Behandlung einer HIV-Infektion bei einem an HIV leidenden Subjekt und/oder zur Verwendung in einem Verfahren zur Behandlung der HIV-Latenz in latent HIV-infizierten Zellen bei einem an HIV leidenden Subjekt.

2. Verbindung zur Verwendung nach Anspruch 1, wobei A ausgewählt ist aus einem Benzimidazol, einem Imidazo[2,1-b]-1,3,4-thiadiazol, einem Benzothiazol, einem Benzoxazolon, einem Purin, Oxazolon, Benzodiazol, Chinazolin, Chinoxalin, Oxazolonpyridin, gegebenenfalls substituiert mit mindestens einem Halogen.

3. Verbindung zur Verwendung nach Anspruch 1 oder 2, wobei A ein Benzimidazol ist, gegebenenfalls mit mindestens einem Halogen, vorzugsweise Chlor und/oder Brom, substituiert.

4. Verbindung zur Verwendung nach einem der vorhergehenden Ansprüche, wobei die Gruppe -Y-[W]n-Z eine Naphthalinoxy-, eine Pyrolmethoxy-, eine Benzyloxy-, eine Benzol- oder eine Phenyloxygruppe darstellt, gegebenenfalls substituiert mit einem oder mehreren ausgewählten Resten ein Halogen und ein C1-C3-Alkyl

5. Verbindung zur Verwendung nach einem der Ansprüche 1 bis 4, wobei R1 ein Methoxy und R2 ein Benzyloxy ist, gegebenenfalls substituiert mit einem Halogen, vorzugsweise einem Chlor,

6. Verbindung zur Verwendung nach einem der Ansprüche 1 bis 4, wobei R1 ein Wasserstoff und R2 ein Benzyloxy ist, gegebenenfalls substituiert mit einem Halogen, vorzugsweise einem Chlor,

7. Verbindung zur Verwendung nach einem der vorhergehenden Ansprüche, wobei diese Verbindung ausgewählt ist aus 4-(Benzyloxy)-N-(5-{imidazo[2,1-b][1,3,4]thiadiazol-2-yl}-2-methoxyphenyl)benzamid, N-[5-(1H-1,3-Benzodiazol-2-yl)-2-methylphenyl]-4-[(4-chlorphenyl)methoxy]-3-methoxybenzamid, N-[4-(1H-1,3-Benzodiazol-2-yl)phenyl]-3-(benzyloxy)benzamid und N-[3-(1H-1,3-Benzodiazol-2-yl)phenyl]-4-[(4-chlorphenyl)methoxy]-3-methoxybenzamid oder Salze davon.

8. Verbindung zur Verwendung nach einem der vorhergehenden Ansprüche, wobei das Verfahren die Verabreichung der Verbindung gemäß Formel (I) zur Reaktivierung von HIV in latent HIV-infizierten Zellen dieses Subjekts umfasst.

9. Verbindung zur Verwendung nach einem der vorhergehenden Ansprüche, wobei die Verbindung parenteral, enteral oder kutan verabreicht wird.

10. Verbindung zur Verwendung nach einem der vorhergehenden Ansprüche, wobei das Verfahren außerdem die Verabreichung eines antiviralen Mittels an dieses Subjekt umfasst.

11. Pharmazeutische Zusammensetzung enthaltend die Verbindung nach einem der Ansprüche 1 bis 7 und einen pharmazeutisch verträglichen Träger.

12. Kit, enthaltend mindestens eine Verbindung nach einem der Ansprüche 1 bis 7 und mindestens ein antivirales Mittel und/oder mindestens ein anderes Latenz-erhöhendes Mittel (LRA), wobei diese Verbindung vorteilhafterweise zur Verwendung in einer Konzentration zwischen 0,03 µM und 15 µM verpackt ist, insbesondere zwischen 3 µM und 10 µM oder zwischen 0,03 µM und 0,1 µM.

13. *In vitro*/*ex vivo* Verwendung einer Verbindung nach einem der Ansprüche 1 bis 7 zur Reaktivierung von HIV in latent HIV-infizierten Zellen.

## Claims

1. A compound according to formula (1), or a salt thereof, 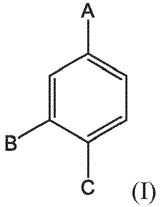 wherein
A represents a heteroaryl optionally substituted by at least one halogen, and
B and C independently represent a radical selected from:
a hydrogen,
a C₁-C₆ alkyl,
a C₁-C₆ alkoxy, and
a group according to formula (II), 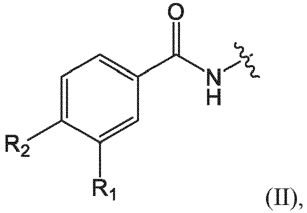
wherein R₁ and R₂ represent, independently of each other, a radical selected from a hydrogen,
a C₁-C₃ alkyl,
a C₁-C₃ alkoxy,
a group according to the formula -Y-[W]n-Z,
wherein
Y is a radical selected from O, N, S and C;
W is a radical selected from C₁-C₆ alkyl, C₁-C₆ alkylene and C₂-C₆ alkenyl;
n is 0 or 1;
Z represents a phenyl, a pyrrole or a naphthalene, aryl, an imidazole, naphthyl, or a phenanthrene optionally substituted by at least one radical selected from a C₁-C₃ alkyl, a C₁-C₃ alkoxy and a halogen
wherein
when C is hydrogen, methyl or methoxy, B is a group according to formula (II), and conversely when B is hydrogen, methyl or methoxy, C is a group according to formula (II), and
when R₁ is hydrogen, C₁-C₃ alkyl or C₁-C₃ alkoxy, R₂ is the group -Y-[W]n-Z, and conversely when R₂ is hydrogen, C₁-C₃ alkyl or C₁-C₃ alkoxy, R₁ is the group -Y-[W]n-Z,
for use in a method for treating HIV infection in a subject with HIV and/or for use in a method for treating HIV latency in latent HIV-infected cells in a subject with HIV.

2. The compound for its use according to claim 1, wherein A is selected from a benzimidazole, an imidazo[2,1-b]-1,3,4-thiadiazole, a benzothiazole, a benzoxazolone, a purine Oxazolone, benzodiazole, quinazoline, quinoxaline, oxazolone pyridine, optionally substituted by at least one halogen.

3. The compound for its use according to claim 1 or 2, wherein A is a benzimidazole optionally substituted by at least one halogen, preferentially a chlorine and/or a bromine.

4. The compound for its use according to one of the preceding claims, wherein the group -Y-[W]n-Z represents a naphthalenoxy, a pyrolmethoxy, a benzyloxy, a benzene or a phenyloxy, optionally substituted by one or more radicals selected from halogen and C₁-C₃ alkyl.

5. The compound for its use according to one of claims 1 to 4, wherein R₁ is methoxy and R₂ is benzyloxy optionally substituted by a halogen, preferably a chlorine.

6. The compound for its use according to one of claims 1 to 4, wherein R₁ is hydrogen and R₂ is benzyloxy optionally substituted by a halogen, preferably a chlorine.

7. The compound for its use according to one of the preceding claims, said compound being selected from 4-(benzyloxy)-N-(5-{imidazo[2,1-b][1,3,4]thiadiazol-2-yl}-2-methoxyphenyl)benzamide, N-[5-(1H-1,3-benzodiazol-2-yl)-2-methylphenyl]-4-[(4-chlorophenyl)methoxy]-3-methoxybenzamide, N-[4-(1H-1,3-benzodiazol-2-yl)phenyl]-3-(benzyloxy)benzamide and N-[3-(1H-1,3-benzodiazol-2-yl)phenyl]-4-[(4-chlorophenyl)methoxy]-3-methoxybenzamide, or salts thereof.

8. The compound for its use according to one of the preceding claims, wherein said method comprises administering the compound according to formula (I) for the reactivation of HIV in latent HIV-infected cells of said subject.

9. The compound for its use according to one of the preceding claims, wherein said compound is administered parenterally, enterally or cutaneously.

10. The compound for use according to one of the preceding claims, wherein the method further comprises administering to said subject an antiviral agent.

11. A pharmaceutical composition comprising the compound according to one of claims 1 to 7 and a pharmaceutically acceptable carrier.

12. A kit comprising at least one compound according to one of claims 1 to 7 and at least one antiviral agent and/or at least one other latency lifting agent (LRA), said compound being advantageously packaged to be used at a concentration comprised between 0.03 µM and 15 µM, in particular between 3 µM and 10 µM or between 0.03 µM and 0.1 µM.

13. An in vitro/ex vivo use of a compound according to one of claims 1 to 7, for the reactivation of HIV in latent HIV-infected cells.
